# EUROPEAN PATENT APPLICATION

(11) **EP 2 316 482 A1**
(43) Date of publication of application: **04.05.2011**
(21) Application number: 10180590.1
(22) Date of filing: 19.03.2002
(51) Int. Cl.: A61K 39/39

(54) **Transcutaneous immunostimulation**

(30) Priority: 19.03.2001 US 276496 P
(62) Divisional of application: 02721446.9
(71) Applicant: Intercell USA, Inc., Gaithersburg, MD 20878 (US)
(72) Inventor: Glenn, Gregory, Gaithersburg, MD 20878 (US); Six, Howard, Lewisburg, TN TN37091 (US)
(74) Representative: Lipscombe, Martin John

(57) **Abstract**

Disclosed is an adjuvant for transcutaneous immunostimulation comprising:
(a) providing a subject in need of immunization with a vaccine,
(b) applying at least one adjuvant epicutaneously to the subject's skin, and
(c) immunizing the subject with the vaccine by a route of administration other than transcutaneous, wherein the vaccine comprises one or more antigens;

whereby the at least one adjuvant causes transcutaneous immunostimulation by inducing an immune response specific for the one or more antigens, wherein the immune response stimulated by the at least one adjuvant is more effective than in the absence of the at least one adjuvant.

## Description

### CROSS-REFERENCE TO RELATED APPLICATIONS

This application claims the benefit of provisional U.S. Appln. No. 60/276,496, filed March 19, 2001.

### FIELD OF THE INVENTION

The invention relates to formulations for transcutaneous immunostimulation; their use to stimulate the immune response induced by a vaccine, to treat disease, to reduce the effective dose of antigen in a vaccine, and combinations thereof, and their manufacture.

### BACKGROUND OF THE INVENTION

A variety of antigens are effectively administered by transcutaneous immunization (TCl) to induce antigen-specific immune responses. See WO 98/20734, WO 99/43350, and WO 00/61184; U.S. Patents 5,910,306 and 5,980,898; and U.S. Patent Applns. 09/257,188; 09/309,881; 09/311,720; 09/316,069; 09/337,746; and 09/545,417. We have previously taught that TCI can be used in conjunction with vaccines administered by other routes (e.g., enteral, mucosal, transdermal, other parenteral) to prime or boost such conventional vaccination. It is now demonstrated that adjuvant alone delivered by transcutaneous immunization can stimulate the immune response in a subject who would otherwise respond poorly, if at all, to conventional vaccination. Transcutaneous immunostimulation may be used in a subject suspected of responding poorly to a conventional vaccination because of age, acquired or congenital immunodeficiency, immunosuppression, or the use of reduced amounts of antigen in the conventional vaccine.

As an example of the application of transcutaneous immunostimulation to conventional vaccines, we have chosen influenza virus and vaccines sold to protect against viral infection. Forms suitable for administration by oral, nasal, or injectable routes may be used as the vaccine. We show that subjects who respond poorly to vaccination may have their antigen-specific immune responses stimulated by transcutaneous delivery of adjuvant. In the context of the present invention, TCI is used to deliver adjuvant for immunostimulation. Adjuvants which may be toxic when administered through other routes can be safely and effectively used transcutaneously. It is advantageous to use an adjuvant capable of stimulating both systemic and mucosal immunity, but the anti-adjuvant immune response may not be essential for treatment. Therefore, it is surprising that an immune response can be orchestrated through transcutaneous delivery of adjuvant and vaccination by an entirely different route.

Glueck et al. (J. Infect. Dis., 181:1129-1132, 2000) used LT as adjuvant for an intranasal influenza vaccine. The vaccine is comprised of both adjuvant and trivalent influenza virosome. Podda (Vaccine, 19:2673-2680, 2001) reviews the use of MF59 as adjuvant for an influenza vaccine administered by intramuscular injection to the elderly. Lu et al. (Vaccine, 20:1019-1029, 2002) used LT or an LT mutant (R192G) as adjuvant for an oral influenza vaccine. Hagiwara et al. (Vaccine, 19:2071-2079, 2001) used LT or an LT mutant (H44A) as adjuvant for an intranasal inactivated viral vaccine.

Chen et al. (J. Virol., 75:7956-7965, 2001) used CT or CpG as adjuvant. The adjuvant is dried to a powder, combined with inactivated monovalent or trivalent influenza vaccine, and injected with a gene gun using a jet. Watabe et al. (Vaccine, 19:4434-4444, 2001) used a cytokine-expressing genetic vector as adjuvant and genetic immunization using a separate expression vector containing the influenza gene encoding M1 matrix protein or M2 membrane antigen.

The aforementioned references neither teach nor suggest separating adjuvant from the vaccine. Removing adjuvant from the vaccine or not including adjuvant in a vaccine, and separately delivering the adjuvant by TCI to stimulate the immune response induced by the vaccine offers the advantage of simplifying the formulation of vaccines and their use. It should also be noted that the above discussion is not an admission that the cited references are prior art because some of them were only recently published.

Furthermore, only a limited supply of influenza vaccine may be available for a population at risk because the combination of serotypes found in the latest offering must be produced in a short period of time to ensure its effectiveness against the current year's most prevalent strains. Therefore, transcutaneous immunostimulation may be used to increase the immunogenic activity of a vaccine having a reduced dose of antigen (*i.e*., dose sparing).

Other advantages of the invention are discussed below or would be apparent from the disclosure herein.

### SUMMARY OF THE INVENTION

It is an object of the invention to stimulate the immune response to a vaccine in a subject. The immune response may be stimulated in a subject who is aged (e.g., over 65 years old), immunodeficient, immunosuppressed, or a combination thereof. Alternatively, the amount of antigen in the vaccine may be reduced.

A formulation containing at least one adjuvant is applied epicutaneously to the subject's skin such that vaccination of the subject is more effective. This is termed "transcutaneous immunostimulation" herein. Separating adjuvant from the antigen used for vaccination allows the adjuvant to be delivered in a safe and effective way, and does not require reformulation of vaccines. Adjuvant preferably targets antigen presenting cells that also process one or more antigens of the vaccine. This does not necessarily require the formulation for TCl to be applied to the site at which the vaccine is delivered, but it may be convenient for the sites at which the formulations are delivered to be the same or adjoining.

The vaccine may contain an insufficient amount of antigen such that an effective immune response is not induced in the absence of adjuvant. Alternatively, the immune response induced by vaccine alone may provide an effective treatment for the subject but immunostimulation with adjuvant can stimulate immune responses that provide a more beneficial treatment (e.g., therapy and/or prophylaxis). Vaccine may be administered by any nontranscutaneous technique: e.g., routes of administration like oral, nasal, and injection. The vaccine may or may not contain adjuvant.

The adjuvant may activate an antigen presenting cell (APC) underlying the skin. APC may migrate to a lymph node. The APC may process and present an immunogenic epitope of an antigen in the vaccine if the antigen is taken up by the APC. The APC (e.g., skin dendritic cell, Langerhans cell) may become activated, migrate to a regional lymph node, and present at least one immunogenic epitope of the antigen. Preferably, the route of administration chosen for immunization with the vaccine intersects with trafficking of an activated APC to the regional lymph node.

The immune response induced by transcutaneous immunostimulation may be enhanced by skin penetration (e.g., chemical, physical). The skin may be hydrated before, after, during, or any combination thereof to enchance the immune response. The antigen-specific immune response may be used to treat a subject (e.g., human, animal) to provide therapy for an existing disease and/or protection from a potential disease. The benefit of the invention may be obtained in at least two different ways:
(i) stimulating an immune response that has not reached a threshold for effective treatment with vaccine alone but achieves the threshold with immunostimulation or
(ii) stimulating an immune response which has reached the threshold for effective treatment but is made more beneficial by immunostimulation.

Effectiveness may be assessed by clinical or laboratory criteria, surrogate markers which are correlated to health, or morbidity or mortality criteria. For example, the benefits of the invention may be shown on a selected population of subjects by epidemiological study. Methods of using and making such formulations are disclosed herein. Further aspects of the invention will be apparent to a person skilled in the art from the following detailed description and claims, and generalizations thereto.

### BRIEF DESCRIPTION OF THE DRAWINGS

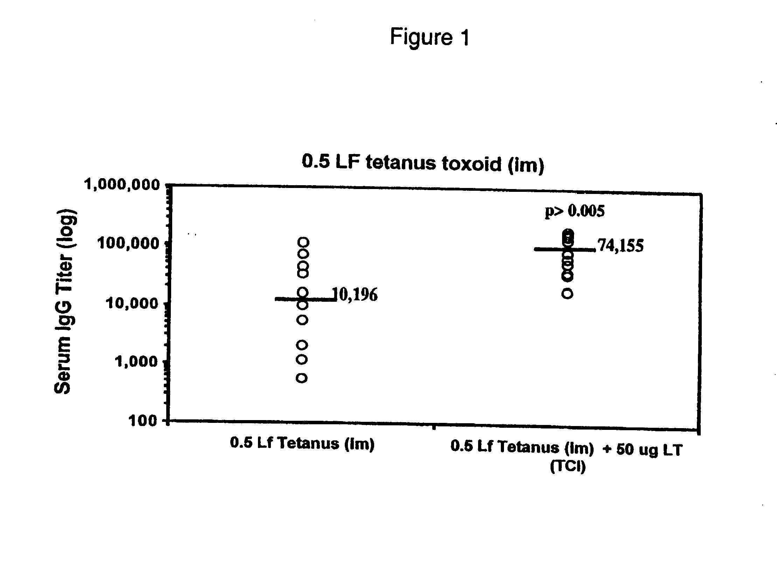

Fig. 1 compares the immune response after intramuscular (im) injection of low-dose tetanus toxoid vaccine with or without epicutaneous application of an adjuvant-containing patch (LT and TCI). Bar indicates the geometric mean titer.

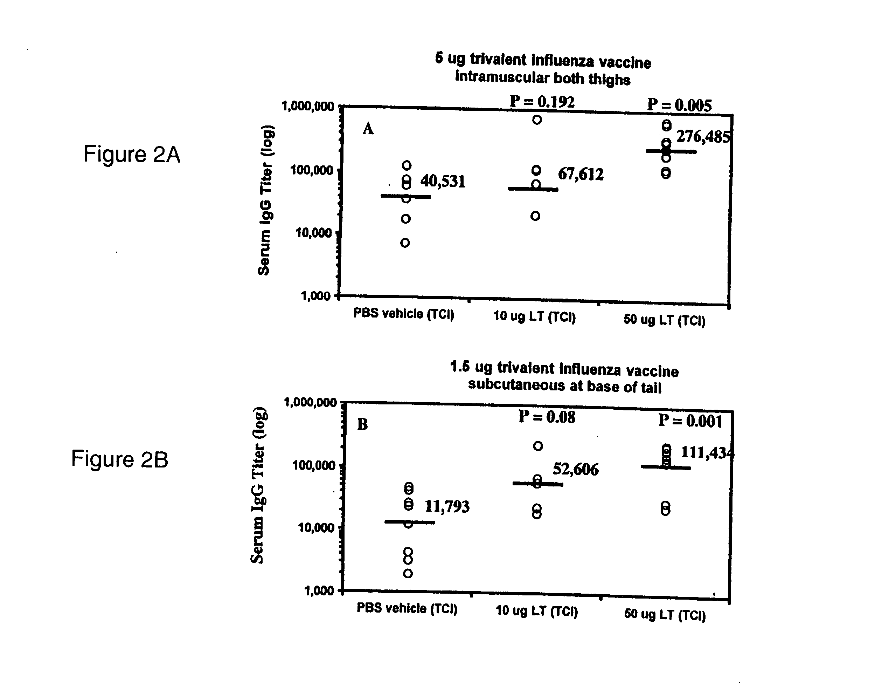

Fig. 2 compares the immune response after parenteral vaccination of low-dose influenza vaccine with or without epicutaneous application of an adjuvant-containing patch (LT and TCl). Fig. 2A shows 5 µg trivalent influenza vaccine injected intramuscularly in both thighs. Fig. 2B shows 1.5 µg trivalent influenza vaccine injected subcutaneously at the base of the tail. Bar indicates the geometric mean titer.

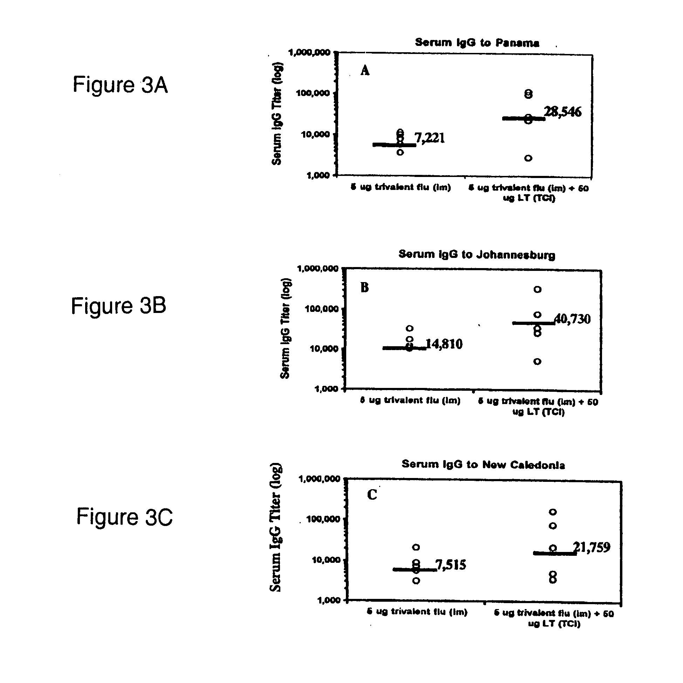

Fig. 3 compares the immune response after intramuscular (im) injection of trivalent influenza vaccine with or without epicutaneous application of an adjuvant-containing patch (LT and TCI). Fig. 3A shows the immune response to Panama A strain. Fig. 3B shows the immune response to Johannesburg B strain. Fig. 3C shows the immune response to New Caledonia A strain. Bar indicates the geometric mean titer.

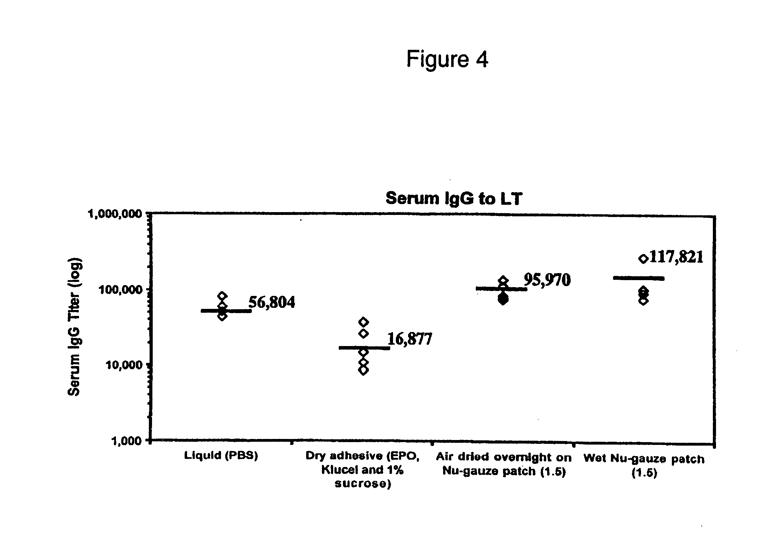

Fig. 4 shows the results from four different formulation strategies that are suitable for transcutaneous delivery of antigen.

### DESCRIPTION OF SPECIFIC EMBODIMENTS OF THE INVENTION

Transcutaneous immunostimulation administers at least one adjuvant by transcutaneous immunization to a subject who has undergone, is undergoing, or will undergo conventional vaccination. A subject is selected for treatment to stimulate the immune response to a conventional vaccine. A suspicion, medical history, or determination by a physician or veterinarian that the subject may fail to respond or only poorly respond to conventional vaccination because of age, acquired or congenital immunodeficiency, immunosuppression caused by disease or ablative therapy, or the use of reduced amounts of antigen in the conventional vaccine can be used to select subjects in need of treatment.

Transcutaneous immunostimulation with an adjuvant can be applied to any vaccine administered orally, into skeletal muscle, or under the skin. Tetanus toxoid and multivalent influenza vaccines are used as examples of the invention. Transcutaneous mmunostimulation can be generally applied to any parenterally administered vaccine. This includes vaccines such as those directed against diphtheria, pertussis, tetanus, hepatitis (A, B and C), rabies, Lyme disease, mumps, measles, influenza virus infection, polyvalent pneumococcal vaccines, yellow fever, *Haemophilus influ*enza infection, rotavirus infection, HIV infection, and malaria infection.

Since certain adjuvants are also potent stimulators of mucoscal immunity, the invention can be applied to vaccines that are traditionally administered directly into mucosal tissues. In this respect, an adjuvant-containing patch would be applied to the skin at the time of oral administration of these vaccines. Examples of this application include vaccines for enteric infections (cholera, shigella, enterotoxigenic E. *coli, Helicobacter pylori,* or attenuated typhoid vaccines). The immunostimulating actions of adjuvant can also be used to enhance the effectiveness of vaccines that are delivered as nasal sprays or by pulmonary inhalation, such as nasal influenza vaccines. This invention is also useful for vaccines that are being developed to prevent or therapeutically treat other respiratory infections by *Bacillus anthracis, Bordetella pertussis, Chlamydia pneumoniae,* Group A and Group B *Streptococci,* rubella, *Moraxella, Pseudomonas,* respiratory syncytial virus, smallpox virus, and *Mycobacteria.* Transcutaneous immunostimulation by adjuvant may also be used with vaccines to prevent or therapeutically treat sexually transmitted diseases caused by *Chlamydia trachomatis, Neisseria gonorrhea,* and *Treponema pallidum.*

The invention also provides a method for stimulating the immune responses in individuals that have acquired immunodeficiency, especially the elderly who become immunocompromised with age (e.g., greater than 65 years old). Adjuvant-containing patches can be used to enhance the immune response to vaccines that are less than effective in the elderly. For example, of the 10,000 to 20,000 people in the US that die each year from influenza, 90% of these deaths are the elderly. The patch used together with influenza vaccination may result in improved mortality and morbidity in the elderly population. The patch may also be useful for individuals with compromised immune systems caused by infectious disease (e.g., HIV) and for individuals undergoing immunosuppressive treatment for cancer and organ transplantation, where opportunistic infections cause a high proportion of deaths.

The development of new vaccines to treat different types of cancer has been hampered by the poor immunogenicity of the cancer vaccines. The invention can be applied to improve the antigenicity of experimental cancer vaccines for preventing or therapeutically treating many cancers (e.g., breast carcinoma, hepatoma, melanoma, prostate carcinoma).

### SKIN STRUCTURE AND IMMUNOBIOLOGY

Skin, the largest human organ, plays an important part in the body's defense against invasion by infectious agents and contact with noxious substances. But this barrier function of the skin appears to have prevented the art from appreciating that transcutaneous immunization provided an effective alternative to enteral, mucosal, and other parenteral routes of administering vaccines. It has recently been shown that epicutaneous application of a vaccine targets specialized antigen presenting cells and induces a robust immune response.

Anatomically, skin is composed of three layers: the epidermis, the dermis, and subcutaneous fat. Epidermis is composed of the basal, the spinous, the granular, and the cornified layers; the stratum corneum comprises the cornified layer and lipid. The principal antigen presenting cells of the skin, Langerhans cells, are reported to be in the mid- to upper-spinous layers of the epidermis in humans. Dermis contains primarily connective tissue. Blood and lymphatic vessels are confined to the dermis and subcutaneous fat.

The stratum corneum, a layer of dead skin cells and lipids, has traditionally been viewed as a barrier to the hostile world, excluding organisms and noxious substances from the viable cells below the stratum corneum. Stratum corneum also serves as a barrier to the loss of moisture from the skin: the relatively dry stratum corneum is reported to have 5% to 15% water content while deeper epidermal and dermal layers are relatively well hydrated with 85% to 90% water content. The barrier function of skin is reinforced by extensive crosslinking between corneocytes. Only recently has the secondary protection provided by antigen presenting cells (e.g., Langerhans cells) been recognized. Moreover, the ability to immunize through the skin with or without penetration enhancement (*i.e.,* transcutaneous immunization) using a skin-active adjuvant has only been recently described. Although undesirable skin reactions such as atopy and dermatitis were known in the art, recognition of the therapeutic advantages of transcutaneous immunization might not have been appreciated in the past because the skin was believed to provide a barrier to the passage of molecules larger than about 500 daltons (Bos et al., Exp. Dermatol., 9:165-169, 2000).

The epidermis is composed primarily of keratinocytes, but also has a significant population (about 1 % to 3%) of immune surveillance cells called Langerhans cells (LC) distributed amongst the viable keratinocytes. Although LC are a relatively small population of cells in the skin, they account for 25% of the total skin surface area in humans. Langerhans cells represent an extensive, superficial network barrier of immune cells that make an attractive target for vaccine delivery. They are bone marrow derived dendritic cells that migrate to epithelial surfaces where they perform immunosurveilance. Under normal circumstances, there is a baseline traffic of LC from the skin to the draining lymph nodes. In the face of a stimulus such as infecting microbes, the number of LC migrating out of the skin is greatly increased, fulfilling the immunosurveillance function of an antigen presenting cell. Langerhans cells stimulated by the danger signals created by interaction with microbes, foreign materials, or adjuvants orchestrate an effector immune response in the lymph node through the highly specific and amplified response created by their antigen presentation function.

A system for transcutaneous immunization (TCI) is provided which induces an immune response (e.g., humoral and/or cellular effector specific for an antigen) in an animal or human. The delivery system provides simple, epicutaneous application of a formulation comprised of at least one adjuvant and/or one or more antigens to the skin of a human or animal subject (Glenn et al., J. Immunol., 161:3211-3214, 1998a; Glenn et al., Nature, 391:851, 1998b; Glenn et al., Nature Med., 6:1403-1406, 2000; Hammond et al., Adv. Drug Deliv. Rev., 43:45-55, 2000; Scharton-Kersten et al., Infect. Immun., 68:5306-5313, 2000). An antigen-specific immune response is thereby elicited with or without chemical and/or physical penetration enhancement as long as the skin is not perforated through the dermal layer. At least one adjuvant may be provided in dry form when administering the formulation and/or as part of a patch. Use of adjuvant stimulates the immune response elicited by a vaccine administered to a subject *(i.e.,* immunostimulation). who is aged, immunodeficient, immunosuppressed, or a combination thereof. This delivery system may also be used in conjunction with enteral, mucosal, or other parenteral immunization techniques. Thus, the technologies described here could be used for treatment of humans and animals such as, for example, immunotherapy and immunoprotection: therapeutically to treat existing disease, protectively to prevent disease, to reduce the severity and/or duration of disease, to ameliorate one or more symptoms of disease, or combinations thereof.

The transit pathways utilized by antigens to traverse the stratum corneum are unknown at this time. The stratum corneum (SC) is the principal barrier to delivery of drugs and antigens through the skin. Transdermal drug delivery of polar drugs is widely held to occur through aqueous intercellular channels formed between the keratinocytes *(*Transdermal and Topical Drug Delivery Systems, Eds. Ghosh et al., Buffalo Grove: Interpharm Press, 1997). Although the SC is the limiting barrier for penetration, it is breached by hair follicles and sweat ducts. Whether antigens penetrate directly through the SC or via the epidermal appendages may depend on a host of factors. These appendages are thought to play only a minor role in transdermal drug delivery (Barry et al., J. Control Rel., 6:85-97, 1987). Despite some evidence in mice that transcutaneous immunization using DNA may utilize hair follicles as the pathway for skin penetration (Fan et al., Nature Biotechnol., 17:870-872, 1999), it is more likely that the robust immune responses utilize more of the skin surface area. Because disruption of the SC barrier can be accomplished by simple hydration of the skin (Roberts et al., In: Pharmaceutical Skin Penetration Enhancement, Eds. Walters et al., New York: Marcel Dekker, 1993), this has been employed for transcutaneous immunization.

Activation of one or more of adjuvant, antigen, and antigen presenting cell (APC) may stimulate the induction of the immune response. The APC processes the antigen and then presents one or more epitopes to a lymphocyte. Activation may promote contact between the formulation and the APC (e.g., Langerhans cells, other dendritic cells, macrophages, B lymphocytes), uptake of the formulation by the APC, processing of antigen and/or presentation of epitopes by the APC, migration and/or differentiation of the APC, interaction between the APC and the lymphocyte, or combinations thereof. The adjuvant by itself may activate the APC. For example, a chemokine may recruit and/or activate antigen presenting cells to a site. In particular, the antigen presenting cell may migrate from the skin to the lymph nodes, and then present antigen to a lymphocyte, thereby inducing an antigen-specific immune response. Furthermore, the formulation may directly contact a lymphocyte which recognizes antigen, thereby inducing an antigen-specific immune response. It is possible that an APC from the skin, stimulated by an adjujvant applied to the skin, may migrate to the draining lymph node and interact with antigen presentation by other APCs that have encountered antigen at another anatomical site such as in muscle tissues and thereby stimulate the immune response to the antigen, such as an antigen delivered by the imntramuscular route. Although the mechanism for how a patch containing an adjuvant placed over the skin in the same draining lymph field might enhance the immune response to a parenterally delivered vaccine given in the same draining lymph field is not known, we demonstrate herein that stimulating the immune response to an intramuscularly delivered vaccine can be accomplished by epicutaneous application of an adjuvant-containing patch.

In addition to eliciting immune reactions leading to activation and/or expansion of antigen-specific B-cell and/or T-cell populations, including antibodies and cytotoxic T lymphocytes (CTL), the invention may positively and/or negatively regulate one or more components of the immune system by using transcutaneous immunization to affect antigen-specific helper (Th1 and/or Th2) or delayed-type hypersensitivity T-cell subsets (T_{DTH}). The desired immune response induced is preferably systemic or regional (e.g., mucosal) but it is usually not undesirable immune responses (e.g., atopy, dermatitis, eczema, psoriasis, and other allergic or hypersensitivity reactions). As seen herein, the immune responses elicited are of the quantity and quality that provide therapeutic or prophylactic immune responses useful for treating disease.

Hydration of the intact or penetrated skin before, during, or immediately after epicutaneous application of the formulation is preferred and may be required in some or many instances. For example, hydration may increase the water content of the topmost layer of skin (e.g., stratum corneum or superficial epidermis layer exposed by penetration enhancement techniques) above 25%, 50% or 75%. Skin may be hydrated with an aqueous solution of 10% glycerol, 70% isopropyl alcohol, and 20% water. Addition of an occlusive dressing or use of a semi-liquid formulation (e.g., cream, emulsion, gel, lotion, paste) can increase hydration of the skin. For example, lipid vesicles or sugars can be added to a formulation to thicken a solution or suspension. Hydration occurs with or without disruption of all or at least a portion of the stratum corneum at the site of application of the formulation, along with possibly also a portion of the epidermis, as long as the dermis is not perforated. The intent is for the formulation to act on skin antigen presenting cells instead of introducing immunologically-active components of the formulation into the systemic circulation, although some portion of the formulation may act at distal sites.

Skin may be swabbed with an applicator (e.g., adsorbent material on a pad or stick) containing hydration or chemical penetration agents or they may be applied directly to skin. For example, aqueous solutions (e.g., water, saline, other buffers), acetone, alcohols (e.g., isopropyl alcohol), detergents (e.g., sodium dodecyl sulfate), depilatory or keratinolytic agents (e.g., calcium hydroxide, salicylic acid, ureas), humectants (e.g., glycerol, other glycols), polymers (e.g., polyethylene or propylene glycol, polyvinyl pyrrolidone), or combinations thereof may be used or incorporated in the formulation. Similarly, abrading the skin (e.g., abrasives like an emery board or paper, sand paper, fibrous pad, pumice), removing a superficial layer of skin (e.g., peeling or stripping with an adhesive tape), microporating the skin using an energy source (e.g., heat, light, sound, electrical, magnetic) or a barrier disruption device (e.g., blade, needle, projectile, spray, tine), or combinations thereof may act as a physical penetration enhancer. See WO 98/29134, WO 01/34185, and WO 02/07813; U.S. Patents 5,445,611, 6,090,790, 6,142,939, 6,168,587, 6,312,612, 6,322,808 and 6,334,856 for description of microblades or microneedles, gun or spray injectors, and for microporation of the skin and techniques that might be adapted for transcutaneous immunization. The objective of chemical or physical penetration enhancement in conjunction with TCI is to remove at least the corneum, or a superficial or deeper epidermal layer, without perforating skin through past the dermal layer. This is preferably accomplished with minor discomfort at most to the human or animal subject, and without bleeding at the site. For example, applying the formulation to intact skin may or may not involve thermal, optical, sonic, or electromagnetic energy to perforate layers of the skin to below the stratum corneum or epidermis.

The difference between transcutaneous immunization as practiced in WO 98/20734 and 99/43350 is whether all or at least a portion of the stratum corneum is disrupted. The term "penetration enhancer" as used herein refers to those chemicals which when applied in the formulation, before application, during application, or after application results in such disruption. Some chemicals (e.g., alcohols) may or may not disrupt the stratum corneum depending on how vigorously they are applied (e.g., swabbing or scrubbing with sufficient pressure). For example, including alcohol, O/W or W/O emulsions, lipid micelles, or lipid vesicles in the formulation may enhance penetration of one or more immunologically-active ingredients of the same formulation across intact skin without detectable disruption of the stratum corneum.

Formulations which are useful for vaccination are also provided as well as processes for their manufacture. The formulation may be in dry or liquid form. A dry formulation is more easily stored and transported than conventional vaccines, it breaks the cold chain required from the vaccine's place of manufacture to the locale where vaccination occurs. Without being limited to any particular mode of action, another way in which a dry formulation may be an improvement over liquid formulations is that high concentrations of a dry active component of the formulation (e.g., one or more adjuvants and/or antigens) may be achieved by solubilization directly at the site of immunization over a short time span. Moisture from the skin (e.g., perspiration) and an occlusive dressing may hasten this process. In this way, it is possible that a concentration approaching the solubility limit of the active ingredient may be achieved *in situ.* Alternatively, the dry, active ingredient of the formulation *per se* may be an improvement by providing a solid particulate form that is taken up and processed by antigen presenting cells. These possible mechanisms are discussed not to limit the scope of the invention or its equivalents, but to provide insight into the operation of the invention and to guide the use of this formulation in immunization and vaccination.

The formulation may be provided as a liquid: cream, emulsion, gel, lotion, ointment, paste, solution, suspension, or other liquid forms. Dry formulations may be provided in various forms: for example, fine or granulated powders, uniform films, pellets, and tablets. The formulation may be dissolved and then dried in a container or on a flat surface (e.g., skin), or it may simply be dusted on the flat surface. It may be air dried, dried with elevated temperature, freeze or spray dried, coated or sprayed on a solid substrate and then dried, dusted on a solid substrate, quickly frozen and then slowly dried under vacuum, or combinations thereof. If different molecules are active ingredients of the formulation, they may be mixed in solution and then dried, or mixed in dry form only.

A "patch" refers to a product which includes a solid substrate (e.g., occlusive or nonocclusive surgical dressing) as well as at least one active ingredient. Liquid may be incorporated in a patch *(i.e.,* a wet patch). One or more active components of the formulation may be applied on the substrate, incorporated in the substrate or adhesive of the patch, or combinations thereof. A liquid formulation may be held in a reservoir or may be mixed with the contents of a reservoir. A dry patch may or may not use a liquid reservoir to solubilize the formulation. Compartments or chambers of the patch may be used to separate active ingredients so that only one of the antigens or adjuvants is kept in dry form prior to administration; separating liquid and solid in this manner allows control over the time and rate of the dissolving of at least one dry, active ingredient.

Formulation in liquid or solid form may be applied with one or more adjuvants and/or antigens both at the same or separate sites or simultaneously or in frequent, repeated applications. The patch may include a controlled-release reservoir or a rate-controlling matrix or membrane may be used which allows stepped release of adjuvant and/or antigen. It may contain a single reservoir with adjuvant and/or antigen, or multiple reservoirs to separate individual antigens and adjuvants. The patch may include additional antigens such that application of the patch induces an immune response to multiple antigens. In such a case, antigens may or may not be derived from the same source, but they will have different chemical structures so as to induce an immune response specific for different antigens. Multiple patches may be applied simultaneously; a single patch may contain multiple reservoirs. For effective treatment, multiple patches may be applied at intervals or constantly over a period of time; they may be applied at different times, for overlapping periods, or simultaneously. At least one adjuvant and/or adjuvant may be maintained in dry form prior to administration. Subsequent release of liquid from a reservoir or entry of liquid into a reservoir containing the dry ingredient of the formulation will at least partially dissolve that ingredient.

Solids (e.g., particles of nanometer or micrometer dimensions) may also be incorporated in the formulation. Solid forms (e.g., nanoparticles or microparticles) may aid in dispersion or solubilization of active ingredients; assist in carrying the formulation through superficial layers of the skin; provide a point of attachment for adjuvant, antigen, or both to a substrate that can be opsonized by antigen presenting cells, or combinations thereof. Prolonged release of the formulation from a porous solid formed as a sheet, rod, or bead acts as a depot.

The formulation may be manufactured under conditions acceptable to appropriate regulatory agencies (e.g., Food and Drug Administration) for biologicals and vaccines. Optionally, components like binders, buffers, colorings, dessicants, diluents, humectants, preservatives, stabilizers, other excipients, adhesives, plasticizers, tackifiers, thickeners, patch materials, or combinations thereof may be included in the formulation even though they are immunologically inactive. They may, however, have other desirable properties or characteristics which improve the effectiveness of the formulation.

A single or unit dose of formulation suitable for administration is provided. The amount of adjuvant or antigen in the unit dose may be anywhere in a broad range from about 0.001 µg to about 10 mg. This range may be from about 0.1 µg to about 1 mg; a narrower range is from about 5 µg to about 500 µg. Other suitable ranges are between about 1 µg and about 10 µg, between about 10 µg and about 50 µg, between about 50 µg and about 200 µg, and between about 1 mg and about 5 mg. A preferred dose for a toxin is about 50 µg or 100 µg or less (e.g., from about 1 µg to about 50 µg or 100 µg). The ratio between antigen and adjuvant may be about 1:1 (e.g., an ADP-ribosylating exotoxin when it is both antigen and adjuvant) but higher ratios may be suitable fcr poor antigens (e.g., about 1:10 or less), or lower ratios of antigen to adjuvant may also be used (e.g., about 10:1 or more).

A formulation comprising adjuvant and antigen or polynucleotide may be applied to skin of a human or animal subject, antigen is presented to immune cells, and an antigen-specific immune response is induced. This may occur before, during, or after infection by pathogen. Only antigen or polynucleotide encoding antigen may be required, but no additional adjuvant, if the immunogenicity of the formulation is sufficient to not require adjuvant activity. The formulation may include an additional antigen such that application of the formulation induces an immune response against multiple antigens (*i.e*., multivalent). In such a case, antigens may or may not be derived from the same source, but the antigens will have different chemical structures so as to induce immune responses specific for the different antigens. Antigen-specific lymphocytes may participate in the immune response and, in the case of participation by B lymphocytes, antigen-specific antibodies may be part of the immune response. The formulations described above may include binders, buffers, colorings, dessicants, diluents, humectants, preservatives, stabilizers, other excipients, adhesives, plasticizers, tackifiers, thickeners, and patch materials known in the art.

The invention is used to treat a subject (e.g., a human or animal in need of treatment such as prevention of disease, protection from effects of infection, therapy of existing disease or symptoms, or combinations thereof). Diseases other than infection include cancer, allergy, and autoimmunity. When the antigen is derived from a pathogen, the treatment may vaccinate the subject against infection by the pathogen or against its pathogenic effects such as those caused by toxin secretion. The invention may be used therapeutically to treat existing disease, protectively to prevent disease, to reduce the severity and/or duration of disease, to ameliorate symptoms of disease, or combinations thereof.

The application site may be protected with anti-inflammatory corticosteroids such as hydrocortisone, triamcinolone and mometazone or nonsteroidal anti-inflammatory drugs (NSAID) to reduce possible local skin reaction or modulate the type of immune response. Similarly, anti-inflammatory steroids or NSAID may be included in the patch material, or liquid or solid formulations; and corticosteroids or NSAID may be applied after immunization. IL-10, TNF-α, other immunomodulators may be used instead of the anti-inflammatory agents. Moreover, the formulation may be applied to skin overlying more than one draining lymph node field using either single or multiple applications. The formulation may include additional antigens such that application induces an immune response to multiple antigens. In such a case, the antigens may or may not be derived from the same source, but the antigens will have different chemical structures so as to induce an immune response specific for the different antigens. Multi-chambered patches could allow more effective delivery of multivalent vaccines as each chamber covers different antigen presenting cells. Thus, antigen presenting cells would encounter only one antigen (with or without adjuvant) and thus would eliminate antigenic competition and thereby enhancing the response to each individual antigen in the multivalent vaccine.

The formulation may be epicutaneously applied to skin to prime or boost the immune response in conjunction with or without penetration techniques, or other routes of immunization. Priming by transcutaneous immunization (TCI) with either single or multiple applications may be followed with enteral, mucosal, transdermal, and/or other parenteral techniques for boosting immunization with the same or altered antigens. Priming by an enteral, mucosal, transdermal, and/or other parenteral route with either single or multiple applications may be followed with transcutaneous techniques for boosting immunization with the same or altered antigens. It should be noted that TCl is distinguished from conventional topical techniques like mucosal or transdermal immunization because the former requires a mucous membrane (e.g., lung, mouth, nose, rectum) not found in the skin and the latter requires perforation of the skin through the dermis. The formulation may include additional antigens such that application to skin induces an immune response to multiple antigens.

In addition to antigen and adjuvant, the formulation may comprise a vehicle. For example, the formulation may comprise an AQUAPHOR, Freund, Ribi, or Syntex emulsion; water-in-oil emulsions (e.g., aqueous creams, ISA-720), oil-in-water emulsions (e.g., oily creams, ISA-51, MF59), microemulsions, anhydrous lipids and oil-in-water emulsions, other types of emulsions; gels, fats, waxes, oil, silicones, and humectants (e.g., glycerol).

Antigen may be derived from any pathogen that infects a human or animal subject (e.g., bacterium, virus, fungus, or protozoan), allergens, and self-antigens. The chemical structure of the antigen may be described as one or more of carbohydrate, fatty acid, and protein (e.g., glycolipid, glycoprotein, lipoprotein). Proteinaceous antigen is preferred. The molecular weight of the antigen may be greater than 500 daltons, 800 daltons, 1000 daltons, 10 kilodaltons, 100 kilodaltons, or 1000 kilodaltons (including intermediate ranges thereof). Chemical or physical penetration enhancement may be preferred for macromolecular structures like cells, viral particles, and molecules of greater than one megadalton, but techniques like hydration and swabbing with a solvent may be sufficient to induce immunization across the skin. Antigen may be obtained by recombinant techniques, chemical synthesis, or at least partial purification from a natural source. It may be a chemical or recombinant conjugates: for example, linkage between chemically reactive groups or protein fusion. Antigen may be provided as a live cell or virus, an attenuated live cell or virus, a killed cell, or an inactivated virus. Alternatively, antigen may be at least partially purified in cell-free form (e.g., cell or viral lysate, membrane or other subcellular fraction). Because most adjuvants would also have immunogenic activity and would be considered antigens, adjuvants would also be expected to have the aforementioned properties and characteristics of antigens. For example, adjuvants and antigens may be prepared using the same techniques (see above).

The choice of adjuvant may allow potentiation or modulation of the immune response. Moreover, selection of a suitable adjuvant may result in the preferential induction of a humoral or cellular immune response, specific antibody isotypes (e.g., IgM, IgD, IgA1, IgA2, IgE, IgG1, IgG2, IgG3, and/or IgG4), and/or specific T-cell subsets (e.g., CTL, Th1, Th2 and/or T_{DTH}). The adjuvant is preferably a chemically activated (e.g., proteolytically digested) or genetically activated (e.g., fusions, deletion or point mutants) ADP-ribosylating exotoxin or B subunit thereof.

An "antigen" is an active component of the formulation which is specifically recognized by the immune system of a human or animal subject after immunization or vaccination. The antigen may comprise a single or multiple immunogenic epitopes recognized by a B-cell receptor *(i.e.,* secreted or membrane-bound antibody) or a T-cell receptor. Proteinaceous epitopes recognized by T-cell receptors have typical lengths and conserved amino acid residues depending on whether they are bound by major histocompatibility complex (MHC) Class I or Class II molecules on the antigen presenting cell. In contrast, proteinaceous epitopes recognized antibody may be of variable length including short, extended oligopeptides and longer, folded polypeptides. Single amino acid differences between epitopes may be distinguished. The antigen may be capable of inducing an immune response against a molecule of a pathogen, allergenic substances, or mammalian host (e.g., autoantigens, cancer antigens, molecules of the immune system). For immunoregulation, that molecule may be an allergen, autoantigen, internal image thereof, or other components of the immune system (e.g., B- or T-cell receptor, co-receptor or ligand thereof, soluble mediator or receptor thereof). Thus, antigen is usually identical or at least derived from the chemical structure of the molecule, but mimetics which are only distantly related to such chemical structures may also be successfully used.

An "adjuvant" is an active component of the formulation to assist in inducing an immune response to the antigen. Adjuvant activity is the ability to increase the immune response to a heterologous antigen *(i.e.,* antigen which is a separate chemical structure from the adjuvant) by inclusion of the adjuvant itself in a formulation or in combination with other components of the formulation or particular immunization techniques. As noted above, a molecule may contain both antigen and adjuvant activities by chemically conjugating antigen and adjuvant or genetically fusing coding regions of antigen and adjuvant; thus, the formulation may contain only one ingredient or component.

The term "effective amount" is meant to describe that amount of adjuvant or antigen which induces an antigen-specific immune response. A "subunit" immunogen or vaccine is a formulation comprised of active components (e.g., adjuvant, antigen) which have been isolated from other cellular or viral components of the pathogen (e.g., membrane or polysaccharide components like endotoxin) by recombinant techniques, chemical synthesis, or at least partial purification from a natural source.

Induction of an immune response may provide treatments of a subject such as, for example, immunoprotection, desensitization, immunosuppression, modulation of autoimmune disease, potentiation of cancer immunosurveillance, prophylactic vaccination to prevent disease, and therapeutic vaccination to ameliorate established disease. A product or method "induces" when its presence or absence causes a statistically significant change in the immune response's magnitude and/or kinetics; change in the induced elements of the immune system (e.g., humoral *vs*. cellular, Th1 vs. Th2); effect on the number and/or the severity of disease symptoms; effect on the health and well-being of the subject (*i.e*., morbidity and mortality); or combinations thereof.

The term "draining lymph node field" as used in the invention means an anatomic area over which the lymph collected is filtered through a set of defined lymph nodes (e.g., cervical, axillary, inguinal, epitrochelear, popliteal, those of the abdomen and thorax). Thus, the same draining lymph node field may be targeted by immunization (e.g., enteral, mucosal, transcutaneous, transdermal, other parenteral,) within the few minutes to days required for antigen presenting cells to migrate to the lymph nodes if the sites and times of immunization are appropriately spaced to bring different components of the formulation together (e.g., two closely located patches with either adjuvant or antigen applied at the same time may be effective when neither alone would be successful). For example, a patch delivering adjuvant by the transcuta-neous technique may be placed on the same arm as is injected with a conventional vaccine to boost its effectiveness in elderly, pediatric, or other immunologically compromised populations. In contrast, applying patches to different limbs may prevent an adjuvant-containing patch from boosting the effectiveness of a patch containing only antigen.

Without being bound to any particular theory for the operation of the invention but only to provide an explanation for our observations, we hypothesize that this transcutaneous delivery system carries antigen to cells of the immune system where an immune response is induced. The antigen may pass through the normally present protective outer layers of the skin *(i.e.,* stratum corneum) and induce the immune response directly, or through an antigen presenting cell population in the epidermis (e.g., macrophage, tissue macrophage, Langerhans cell, other dendritic cells, B lymphocyte, or Kupffer cell) that presents processed antigen to lymphocytes. Thus, with or without penetration enhancement techniques, the dermis is not penetrated for TCl as it is for subcutaneous injection or transdermal techniques. Optionally, the antigen may pass through the stratum corneum via a hair follicle or a skin organelle (e.g., sweat gland, oil gland).

Transcutaneous immunization with bacterial ADP-ribosylating exotoxins (bARE) as an example, may target the epidermal Langerhans cell, known to be among the most efficient of the antigen presenting cells (APC). Maturation of APC may be assessed by morphology and phenotype (e.g., expression of MHC Class II molecules, CD83, or co-stimulatory molecules). We have found that bARE appear to activate Langerhans cells when applied epicutaneously to intact skin. Adjuvants such as trypsin-cleaved bARE may enhance Langerhans cell activation. Langerhans cells direct specific immune responses through phagocytosis of the antigens, and migration to the lymph nodes where they act as APC to present the antigen to lymphocytes, and thereby induce a potent antibody response. Although the skin is generally considered a barrier to pathogens, the imperfection of this barrier is attested to by the numerous Langerhans cells distributed throughout the epidermis that are designed to orchestrate the immune response against organisms invading through the skin. According to Udey (Clin. Exp. Immunol., 107:s6-s8, 1997):
Langerhans cells are bone-marrow derived cells that are present in all mammalian stratified squamous epithelia. They comprise all of the accessory cell activity that is present in uninflamed epidermis, and in the current paradigm are essential for the initiation and propagation of immune responses directed against epicutaneously applied antigens. Langerhans cells are members of a family of potent accessory cells ('dendritic cells') that are widely distributed, but infrequently represented, in epithelia and solid organs as well as in lymphoid tissue.
It is now recognized that Langerhans cells (and presumably other dendritic cells) have a life cycle with at least two distinct stages. Langerhans cells that are located in epidermis constitute a regular network of antigen-trapping 'sentinel' cells. Epidermal Langerhans cells can ingest particulates, including microorganisms, and are efficient processors of complex antigens. However, they express only low levels of MHC class I and II antigens and costimulatory molecules (ICAM-1, B7-1 and B7-2) and are poor stimulators of unprimed T cells. After contact with antigen, some Langerhans cells become activated, exit the epidermis and migrate to T-cell-dependent regions of regional lymph nodes where they localize as mature dendritic cells. In the course of exiting the epidermis and migrating to lymph nodes, antigen-bearing epidermal Langerhans cells (now the 'messengers') exhibit dramatic changes in morphology, surface phenotype and function. In contrast to epidermal Langerhans cells, lymphoid dendritic cells are essentially non-phagocytic and process protein antigens inefficiently, but express high levels of MHC class I and class II antigens and various costimulatory molecules and are the most potent stimulators of naive T cells that have been identified."

The potent antigen presenting capability of Langerhans cells can be exploited for transcutaneously-delivered immunogens and vaccines. An immune response using the skin's immune system may be achieved by delivering the formulation only to Langerhans cells in the stratum corneum (*i.e.,* the outermost layer of the skin consisting of cornified cells and lipids) and subsequently activating the Langerhans cells to take up antigen, migrate to B-cell follicles and/or T-cell dependent regions, and present the antigen to B and/or T lymphocytes. If antigens other that bARE (e.g., toxin, colonization or virulence factor) are to be phagocytosed by Langerhans cells, then these antigens could also be transported to the lymph node for presentation to T lymphocytes and subsequently induce an immune response specific for that antigen. Thus, a feature of TCI is the activation of the Langerhans cell, presumably by bARE or derivatives thereof, chemokines, cytokines, PAMP, or other Langerhans cell activating substance including contact sensitizers and adjuvants. Increasing the size of the skin population of Langerhans cells or their state of activation would also be expected to enhance the immune response (e.g., acetone pretreatment). In aged subjects or Langerhans cell-depleted skin *(i.e*., from UV damage), it may be possible to replenish the population of Langerhans cells (e.g., tretinoin pretreatment).

Adjuvants such as bARE are known to be highly toxic when injected or given systemically. But if placed on the surface of intact skin (*i.e.,* epicutaneous), they are unlikely to induce systemic toxicity. Thus, the transcutaneous route may allow the advantage of adjuvant effects without systemic toxicity. A similar absence of toxicity could be expected if the skin were penetrated only below the stratum corneum (e.g., near or at the epidermis), but not through the dermis. Thus, the ability to induce activation of the immune system through the skin induces potent immune responses without systemic toxicity.

The magnitude of the antibody response induced by affinity maturation and isotype switching to predominantly IgG antibodies is generally achieved with T-cell help, and activation of both Th1 and Th2 pathways is suggested by the production of IgG1 and IgG2a. Alternatively, a large antibody response may be induced by a thymus-independent antigen type 1 (Tl-1 ) which directly activates the B lymphocyte or could have similar activating effects on B lymphocytes such as up-regulation of MHC Class II, CD25, CD40, B7-1/CD80, B7-2/CD86, and ICAM-1 molecules.

The spectrum of commonly known skin immune responses is represented by atopy and contact dermatitis. Contact dermatitis, a pathogenic manifestation of Langerhans cell activation, is directed by Langerhans cells which phagocytose antigen, migrate to lymph nodes, present antigen, and sensitize T lymphocytes that migrate to the skin and cause the intense destructive cellular response that occurs at affected skin sites. Such responses are not generally known to be associated with antigen-specific IgG antibodies. Atopic dermatitis may utilize the Langerhans cell in a similar fashion, but is identified with Th2 cells and is generally associated with high levels of IgE antibody.

On the other hand, transcutaneous immunization with bARE provides a useful and desirable immune response. There are usually no findings typical of atopy or contact dermatitis given the high levels of IgG that are induced. Cholera toxin or *E. coli* heat-labile enterotoxin epicutaneously applied to skin can achieve immunization in the absence of lymphocyte infiltration 24, 48 and 120 hours after immunization. The minor skin reactivity seen in preclinical trials were easily treated. This indicates that Langerhans cells engaged by transcutaneous immunization as they "comprise all of the accessory cell activity that is present in uninflamed epidermis, and in the current paradigm are essential for the initiation and propagation of immune responses directed against epicutaneously applied antigens" (Udey, 1997). The uniqueness of the transcutaneous immune response here is also indicated by both the high levels of antigen-specific IgG antibody and the type of antibody produced (e.g., IgM, IgG1, IgG2a, IgG2b, IgG3 and IgA), and generally the absence of antigen specific IgE antibody. Transcutaneous immunization could conceivably occur in tandem with skin inflammation if sufficient activation of antigen presenting cells and T lymphocytes were to occur in a transcutaneous response coexisting with atopy or contact dermatitis.

Transcutaneous targeting of Langerhans cells may also be used in tandem with agents to deactivate all or part of their antigen presenting function, thereby modifying immunization or preventing sensitization. Techniques to modulate Langerhans activation or other skin immune cells include, for example, the use of anti-inflammatory steroidal or nonsteroidal agents (NSAID); cyclosporin, FK506, rapamycin, cyclophosphamide, glucocorticoids, or other immunosuppressants; interleukin-10; interleukin-1 monoclonal antibodies (mAB) or soluble receptor antagonists (RA); interleukin-1 converting enzyme (ICE) inhibitors; or depletion via superantigens such as through *Staphylococcal* enterotoxin A (SEA) induced epidermal Langerhans cell depletion. Similar compounds may be used to modify the innate response of Langerhans cells and induce different T-helper responses (Th1 or Th2) or may modulate skin inflammatory responses to decrease potential side effects of the immunization. Similarly, lymphocytes may be immunosuppressed before, during or after immunization by administering immunosuppressant separately or by coadministration of immunosuppressant with the formulation. For example, it may be possible to induce a potent systemic protective immune responses with agents that would normally result in allergic or irritant contact hypersensitivity but adding inhibitors of ICE may alleviate adverse skin reactions.

### ANTIGEN

A transcutaneous immunization system delivers agents to specialized cells (e.g., antigen presentation cell, lymphocyte) that produce an immune response. These agents as a class are called antigens. Antigen may be composed of chemical structures such as, for example, carbohydrate, glycolipid, glycoprotein, lipid, lipoprotein, phospholipid, polypeptide, conjugates thereof, or any other material known to induce an immune response. Antigen may be conjugated to carrier. Antigen may be provided as a whole organism such as, for example, a bacterium or virion; antigen may be obtained from an extract or lysate, either from whole cells or membrane alone; or antigen may be chemically synthesized or produced by recombinant technology. Antigen may be incorporated into a formulation by solubilization or dispersion.

Antigen of the invention may be expressed by recombinant technology, preferably as a fusion with an affinity or epitope tag; chemical synthesis of an oligopeptide, either free or conjugated to carrier proteins, may be used to obtain antigen of the invention. Oligopeptides are considered a type of polypeptide. Oligopeptide lengths of 6 residues to 20 residues are preferred. Polypeptides may also by synthesized as branched structures. Antigenic polypeptides include, for example, synthetic or recombinant B-cell and T-cell epitopes, universal T-cell epitopes, and mixed T-cell epitopes from one organism or disease and B-cell epitopes from another. Antigen obtained through recombinant technology or peptide synthesis, as well as antigen obtained from natural sources or extracts, may be purified by the antigen's physical and chemical characteristics, preferably by fractionation or chromatography. Recombinants may combine antigen fragments or fuse them into chimerae. A multivalent antigen formulation may be used to induce an immune response to more than one antigen at the same time. Conjugates may be used to induce an immune response to multiple antigens, to boost the immune response, or both. Transcutaneous immunization may be used to boost responses induced initially by other routes of immunization such as by oral, nasal or other parenteral routes. Such oral/transcutaneous or transcutaneous/oral immunization may be especially important to enhance mucosal immunity in diseases where mucosal immunity correlates with protection.

Antigen may be solubilized in a buffer or water or organic solvents such as alcohol or DMSO, or incorporated in gels, emulsions, lipid micelles or vesicles, and creams. Suitable buffers include, but are not limited to, phosphate buffered saline Ca⁺⁺/Mg⁺⁺ free, phosphate buffered saline, normal saline (150 mM NaCl in water), and Hepes or Tris buffer. Antigen not soluble in neutral buffer can be solubilized in 10 mM acetic acid and then diluted to the desired volume with a neutral buffer such as PBS. In the case of antigen soluble only at acid pH, acetate-PBS at acid pH may be used as a diluent after solubilization in dilute acetic acid. Dimethyl sulfoxide and glycerol may be suitable nonaqueous buffers for use in the invention.

A hydrophobic antigen can be solubilized in a detergent or surfactant, for example a polypeptide containing a membrane-spanning domain. Furthermore, for formulations containing liposomes, an antigen in a detergent solution (e.g., cell membrane extract) may be mixed with lipids, and liposomes then may be formed by removal of the detergent by dilution, dialysis, or column chromatography. Certain antigens (e.g., membrane proteins) need not be soluble *per se,* but can be inserted directly into a lipid membrane (e.g., virosome), in a suspension of virion alone, or suspensions of microspheres or heat-inactivated bacteria which may be taken up by activate antigen presenting cells (e.g., opsonization). Antigens may also be mixed with a penetration enhancer as described in WO 99/43350.

Many antigens are known in the art which can be used to vaccinate human or animal subjects and induce an immune response specific for particular pathogens, as well as methods of preparing antigen, determining a suitable dose of antigen, assaying for induction of an immune response, and treating infection by a pathogen (e.g., bacterium, virus, fungus, or protozoan). Allergens and self-antigens of the mammalian host (e.g., human, animal) are examples of antigens that are not derived from a pathogen. Antigen used to produce formulations and vaccines for transcutaneous immunization may be the natural product *per* se, genetically-engineered or chemically-synthesized forms thereof, fragments thereof, fusions, or conjugates. The immune response will usually recognize only a portion of the antigen (e.g., one or more immunogenic epitopes).

Plotkin and Mortimer (Vaccine, 2nd Ed., Philadelphia: W.B. Saunders, 1994) provide antigens which can be used to vaccinate humans or animals to induce an immune response specific for particular pathogens, as well as methods of preparing antigen, determining a suitable dose of antigen, assaying for induction of an immune response, and treating infection by a pathogen.

Bacteria include, for example: anthrax, *Campylobacter, Vibrio cholera,* clostridia including *Clostridium difficile, Diphtheria,* enterohemorrhagic E. *coli,* enterotoxigenic E. *coli, Giardia,* gonococcus, *Helicobacter pylori, Hemophilus influenza B, Hemophilus influenza* nontypeable, *Legionella,* meningococcus, *Mycobacteria* including those organisms responsible for tuberculosis, pertussis, pneumococcus, salmonella, shigella, staphylococcus, Group A beta-hemolytic streptococcus, Streptococcus B, tetanus, *Borrelia burgdorfi* and *Yersinia.* Products thereof which may be used as antigen. Antigen includes, for example, toxins, toxoids, subunits thereof, or combinations thereof; virulence or colonization factors; and products.

Viruses include, for example: adenovirus, dengue serotypes 1 to 4, ebola, enterovirus, hanta virus, hepatitis serotypes A to E, herpes simplex virus 1 or 2, human immunodefi-ciency virus, human papilloma virus, influenza, measles, Norwalk, Japanese equine encephalitis, papilloma virus, parvovirus B19, polio, rabies, respiratory syncytial virus, rotavirus, rubella, rubeola, St. Louis encephalitis, vaccinia, viral expression vectors containing genes coding for other antigens such as malaria antigens, varicella, and yellow fever. The viral products or derivatives thereof may be used as sources for antigen.

Fungi including entities responsible for tinea corporis, tinea unguis, sporotrichosis, aspergillosis, candida and other pathogenic fungi. The fungal products or derivatives thereof may be used as sources for antigen.

Protozoans include, for example: *Entamoeba histolytica, Plasmodium, Leishmania,* and the *Helminthes; Schistosomes;* and products thereof. The protozoan products or derivatives thereof may be used as sources for antigen.

Of particular interest are pathogens that enter on or through mucosal surfaces such as, for example, pathogenic species in the bacterial genera *Actinomyces, Aeromonas, Bacillus, Bacteroides, Bordetella, Brucella, Campylobacter, Capnocytophaga, Clamydia, Clostridium, Corynebacterium, Eikenella, Erysipelothrix, Escherichia, Fusobacterium, Hemophilus, Klebsiella, Legionella, Leptospira, Listeria, Mycobacterium, Mycoplasma, Neisseria, Nocardia, Pasteurella, Proteus, Pseudomonas, Rickettsia, Salmonella, Selenomonas, Shigella, Staphylococcus, Streptococcus, Treponema, Vibrio,* and *Versinia;* pathogenic viral strains from the groups *Adenovirus, Coronavirus, Herpesvirus, Orthomyxovirus, Picornovirus, Poxvirus, Reovirus, Retrovirus, Rotavirus;* pathogenic fungi from the genera *Aspergillus, Blastomyces, Candida, Coccidiodes, Cryptococcus, Histoplasma,* and *Phycomyces;* and pathogenic protozoans in the genera *Eimeria, Entamoeba, Giardia,* and *Trichomonas.*

Vaccination has also been used as a treatment for cancer, allergies, and autoimmune disease. For example, vaccination with tumor antigen (e.g., HER2, prostate specific antigen) may induce an immune response in the form of antibodies, CTLs and lymphocyte proliferation which allows the body's immune system to recognize and kill tumor cells. Tumor antigens useful for vaccination have been described for leukemia, lymphoma, and melanoma. Allergens are known for animals (e.g., bird, cat, dog, rodents), cockroaches, fleas, mites, and plant pollen (e.g., grasses, trees). Vaccination with T-cell receptor or autoantigens (e.g., pancreatic islet antigen) may induce an immune response that halts progression of autoimmune disease.

### ADJUVANT

The formulation contains an adjuvant, although a single molecule may contain both adjuvant and antigen properties (e.g., ADP-ribosylating exotoxin). Because most adjuvants would also have immunogenic activity and would be considered antigens, adjuvants would also be expected to have the aforementioned properties and characteristics of antigens. For example, adjuvants and antigens may be prepared using the same techniques (see above).

Adjuvants are substances that are used to specifically or nonspecifically potentiate an antigen-specific immune response, perhaps through activation of antigen presenting cells (e.g., dendritic cells in various layers of the skin, especially Langerhans cells). See also Elson et al. (in Handbook of Mucosal Immunology, Academic Press, 1994). Although activation may initially occur in the epidermis or dermis, the effects may persist as the dendritic cells migrate through the lymph system and the circulation. Adjuvant may be formulated and applied with or without antigen, but generally, activation of antigen presenting cells by adjuvant occurs prior to presentation of antigen. Alternatively, they may be separately presented within a short interval of time but targeting the same anatomical region (e.g., the same draining lymph node field).

Adjuvants include, for example, chemokines (e.g., defensins, HCC-1, HCC-4, MCP-1, MCP-3, MCP-4, MIP-1α, MIP-1β, MIP-1δ, MIP-3α, MIP-2, RANTES); other ligands of chemokine receptors (e.g., CCR1, CCR-2, CCR-5, CCR-6, CXCR-1); cytokines (e.g., IL-1β, IL-2, IL-6, IL-8, IL-10, IL-12; IFN-γ; TNF-α; GM-CSF); other ligands of receptors for those cytokines, immunostimulatory CpG motifs in bacterial DNA or oligonucleotides; muramyl dipeptide (MDP) and derivatives thereof (e.g., murabutide, threonyl-MDP, muramyl tripeptide); heat shock proteins and derivatives thereof; *Leishmania* homologs of elF4a and derivatives thereof; bacterial ADP-ribosylating exotoxins and derivatives thereof (e.g., genetic mutants, A and/or B subunit-containing fragments, chemically toxoided versions); chemical conjugates or genetic recombinants containing bacterial ADP-ribosylating exotoxins or derivatives thereof; C3d tandem array; lipid A and derivatives thereof (e.g., monophosphoryl or diphosphoryl lipid A, lipid A analogs, AGP, AS02, AS04, DC-Chol, Detox, OM-174); ISCOMS and saponins (e.g., Quil A, QS-21); squalene; superantigens; or salts (e.g., aluminum hydroxide or phosphate, calcium phosphate). See also Nohria et al. (Biotherapy, 7:261-269, 1994) and Richards et al. (in Vaccine Design, Eds. Powell et al., Plenum Press, 1995) for other useful adjuvants.

Adjuvant may be chosen to preferentially induce antibody or cellular effectors, specific antibody isotypes (e.g., IgM, IgD, IgA1, IgA2, secretory IgA, IgE, IgG1, IgG2, IgG3, and/or IgG4), or specific T-cell subsets (e.g., CTL, Th1, Th2 and/or T_{DTH}). For example, antigen presenting cells may present Class II-restricted antigen to precursor CD4+ T cells, and the Th1 or Th2 pathway may be entered. T helper cells actively secreting cytokine are primary effector cells; they are memory cells if they are resting. Reactivation of memory cells produces memory effector cells. Th1 characteristically secrete IFN-γ (TNF-β and IL-2 may also be secreted) and are associated with "help" for cellular immunity, while Th2 characteristically secrete IL-4 (IL-5 and IL-13 may also be secreted) and are associated with "help" for humoral immunity. Depending on disease pathology, adjuvants may be chosen to prefer a Th1 response (e.g., antigen-specific cytolytic cells) *vs.* a Th2 response (e.g., antigen-specific antibodies).

Unmethylated CpG dinucleotides or similar motifs are known to activate B lymphocytes and macrophages (see U.S. Patent 6,218,371). Other forms of bacterial DNA can be used as adjuvants. Bacterial DNA is among a class of structures which have patterns allowing the immune system to recognize their pathogenic origins to stimulate the innate immune response leading to adaptive immune responses. These structures are called pathogen-associated molecular patterns (PAMP) and include lipopolysaccharides, teichoic acids, unmethylated CpG motifs, doublestranded RNA, and mannins. PAMP induce endogenous signals that can mediate the inflammatory response, act as costimulators of T-cell function and control the effector function. The ability of PAMP to induce these responses play a role in their potential as adjuvants and their targets are antigen presenting cells such as dendritic cells and macrophages. The antigen presenting cells of the skin could likewise be stimulated by PAMP transmitted through the skin. For example, Langerhans cells, a type of dendritic cell, could be activated by PAMP in solution on the skin with a transcutaneously poorly immunogenic molecule and be induced to migrate and present this poorly immunogenic molecule to T-cells in the lymph node, inducing an antibody response to the poorly immunogenic molecule. PAMP could also be used in conjunction with other skin adjuvants such as cholera toxin to induce different costimulatory molecules and control different effector functions to guide the immune response, for example from a Th2 to a Th1 response.

Most ADP-ribosylating exotoxins (bARE) are organized as A: B heterodimers with a B subunit containing the receptor binding activity and an A subunit containing the ADP-ribosyltransferase activity. Exemplary bARE include cholera toxin (CT) *E. coli* heat-labile enterotoxin (LT), diphtheria toxin, *Pseudomonas* exotoxin A (ETA), pertussis toxin (PT), C. *botulinum* toxin C2, C. *botulinum* toxin C3, C. *limosum* exoenzyme, *B. cereus* exoenzyme, *Pseudomonas* exotoxin S, S. *aureus* EDIN, and *B. sphaericus* toxin. Mutant bARE, for example containing mutations of the trypsin cleavage site (e.g., Dickenson et al., Infect Immun, 63:1617-1623, 1995) or mutations affecting ADP-ribosylation (e.g., Douce et al., Infect Immun, 65:28221-282218, 1997) may be used.

TCI may be accompished through the ganglioside GM₁, binding activity of CT, LT, or subunits thereof (e.g., CTB or LTB). Ganglioside GM₁ is a ubiquitous cell membrane glycolipid found in all mammalian cells. When the pentameric CT B subunit binds to the cell surface, a hydrophilic pore is formed which allows the A subunit to insert across the lipid bilayer. Other binding targets on the APC may be utilized (e.g., ETA binds α₂-macroglobulin receptor-low density lipoprotein receptor-related protein). The LT B subunit binds to ganglioside GM₁ in addition to other gangliosides and its binding activities may account for its the fact that LT is highly immunogenic on the skin.

TCI with bARE or B subunit-containing fragments or conjugates thereof may require their ganglioside GM₁ binding activity. When mice were transcutaneously immunized with CT, CTA and CTB, CT and CTB were required for induction of an immune response. CTA contains the ADP-ribosylating exotoxin activity but only CT and CTB containing the binding activity are able to induce an immune response indicating that the B subunit was necessary and sufficient to immunize through the skin. We conclude that the Langerhans cells or other APC may be activated by CTB binding to its cell surface resulting in a transcutaneous immune response.

CT, LT, ETA and PT, despite having different cellular binding sites, are potent adjuvants for transcutaneous immunization, inducing IgG antibodies but not IgE antibodies. CTB without CT can also induce IgG antibodies. Thus, both bARE and a derivative thereof can effectively immunize when epicutaneously applied to the skin. Native LT as an adjuvant and antigen, however, is clearly not as potent as native CT. But activated bARE can act as adjuvants for weakly immunogenic antigens in a transcutaneous immunization system. Thus, therapeutic immunization with one or more antigens could be used separately or in conjunction with immunostimulation of the antigen presenting cell to induce a prophylactic or therapeutic immune response.

In general, toxins can be chemically inactivated to form toxoids which are less toxic but remain immunogenic. We envision that the transcutaneous immunization system using toxin-based immunogens and adjuvants can achieve anti-toxin levels adequate for protection against these diseases. The anti-toxin antibodies may be induced through immunization with the toxins, or genetically-detoxified toxoids themselves, or with toxoids and adjuvants. Genetically toxoided toxins which have altered ADP-ribosylating exotoxin activity or trypsin cleavage site, but not binding activity, are envisioned to be especially useful as nontoxic activators of antigen presenting cells used in transcutaneous immunization and may reduce concerns over toxin use.

bARE can also act as an adjuvant to induce antigen-specific CTL through transcutaneous immunization. The bARE adjuvant may be chemically conjugated to other antigens including, for example, carbohydrates, polypeptides, glycolipids, and glycoprotein antigens. Chemical conjugation with toxins, their subunits, or toxoids with these antigens would be expected to enhance the immune response to these antigens when applied epicutaneously. To overcome the problem of the toxicity of the toxins (e.g., diphtheria toxin is known to be so toxic that one molecule can kill a cell) and to overcome the problems of working with such potent toxins as tetanus, several workers have taken a recombinant approach to producing genetically-produced toxoids. This is based on inactivating the catalytic activity of the ADP-ribosyl transferase by genetic deletion. These toxins retain the binding capabilities, but lack the toxicity, of the natural toxins. Such genetically toxoided exotoxins would be expected to induce a transcutaneous immune response and to act as adjuvants. They may provide an advantage in a transcutaneous immunization system in that they would not create a safety concern as the toxoids would not be considered toxic. Activation through a technique such as trypsin cleavage, however, would be expected to enhance the adjuvant qualities of LT through the skin which lacks trypsin-like enzymes. Additionally, several techniques exist to chemically modify toxins and can address the same problem. These techniques could be important for certain applications, especially pediatric applications, in which ingested toxins might possibly elicit adverse reactions.

Adjuvant may be biochemically purified from a natural source (e.g., pCT or pLT) or recombinantly produced (e.g., rCT or rLT). ADP-ribosylating exotoxin may be purified either before or after proteolysis (*i.e*., activation). B subunit of the ADP-ribosylating exotoxin may also be used: purified from the native enzyme after proteolysis or produced from a fragment of the entire coding region of the enzyme. The subunit of the ADP-ribosylating exotoxin may be used separately (e.g., CTB or LTB) or together (e.g., CTA-LTB, LTA-CTB) by chemical conjugation or genetic fusion. A fragment of the ADP-ribosylating exotoxin which retains the ability to bind its cell membrane receptor may also be biochemically purified or recombinantly produced, and then used instead of the B subunit.

Point mutations (e.g., single, double, or triple amino acid substitutions), deletions (e.g., protease recognition site), and isolated functional domains of ADP-ribosylating exotoxin may also be used as adjuvant. Derivatives which are less toxic or have lost their ADP-ribosylation activity, but retain their adjuvant activity have been described. Specific mutants of *E. coli* heat-labile enterotoxin include LT-K63, LT-R72, LT (H44A), LT (R192G), LT (R192G/L211A), and LT (Δ192-194). Toxicity may be assayed with the Y-1 adrenal cell assay (Clements and Finkelstein, Infect. Immun., 24:760-769, 1979). ADP-ribosylation may be assayed with the NAD-agmatine ADP-ribosyltransferase assay (Moss et al., J. Biol. Chem., 268:6383-6387, 1993). Particular ADP-ribosylating exotoxins, derivatives thereof, and processes for their production and characterization are described in U.S. Patents 4,666,837; 4,935,364; 5,308,835; 5,785,971; 6,019,982; 6,033,673; and 6,149,919.

An activator of Langerhans cells may also be used as an adjuvant. Examples of such activators include: inducers of heat shock protein; contact sensitizers (e.g., trinitrochlorobenzene, dinitrofluorobenzene, nitrogen mustard, pentadecylcatechol); toxins (e.g., Shiga toxin, Staph enterotoxin B); lipopolysaccharide (LPS), lipid A, or derivatives thereof; bacterial DNA; chemokines, cytokines, differentiation factors, or growth factors (e.g., members of the TGFβ superfamily); and extracellular calcium or calcium ionophores that increase intracellular [Ca⁺⁺]. See U.S. Patent 6,210,672.

If an immunizing antigen has sufficient Langerhans cell activating capabilities then a separate adjuvant may not be required, as in the case of LT which is both antigen and adjuvant. Alternatively, such antigens can be considered not to require an adjuvant because they are sufficiently immunogenic. It is envisioned that live cell or virus preparations, attenuated live cells or viruses, killed cells, inactivated viruses, and DNA plasmids could be effectively used for transcutaneous immunization. It may also be possible to use low concentrations of contact sensitizers or other activators of Langerhans cells to induce an immune response without inducing skin lesions.

Other techniques for enhancing activity of adjuvants may be effective, such as adding surfactants and/or phospholipids to the formulation to enhance adjuvant activity of ADP-ribosylating exotoxin by ADP-ribosylation factor. One or more ADP-ribosylation factors (ARF) may be used to enhance the adjuvanticity of bARE (e.g., ARF1, ARF2, ARF3, ARF4, ARF5, ARF6, ARD1). Similarly, one or more ARF could be used with an ADP-ribosylating exotoxin to enhance its adjuvant activity.

Undesirable properties or harmful side effects (e.g., allergic or hypersensitive reaction; atopy, contact dermatitis, or eczema; systemic toxicity) may be reduced by modification without destroying its effectiveness in transcutaneous immunization. Modification may involve, for example, removal of a reversible chemical modification (e.g., proteolysis) or encapsulation in a coating which reversibly isolates one or more components of the formulation from the immune system. For example, one or more components of the formulation may be encapsulated in a particle for delivery (e.g., microspheres, nanoparticles) although we have shown that encapsulation in lipid vesicles is not required for transcutaneous immunization and appears to have a negative effect. Phagocytosis of a particle may, by itself, enhance activation of an antigen presenting cell by upregulating expression of MHC Class I and/or Class II molecules and/or costimulatory molecules (e.g., CD40, B7 family members like CD80 and CD86). Alternative methods of upregulating such molecules by activating an antigen presenting cell are also known (see above).

### FORMULATION

Processes for manufacturing a pharmaceutical formulation are well known. The components of the formulation may be combined with a pharmaceutically-acceptable carrier or vehicle, as well as any combination of optional additives (e.g., at least one binder, buffer, coloring, dessicant, diluent, humectant, preservative, stabilizer, other excipient, or combinations thereof). See, generally, Ullmann's Encyclopedia of Industrial Chemistry, 6th Ed. (electronic edition, 1998); Remington's Pharmaceutical Sciences, 22nd (Gennaro, 1990, Mack Publishing); Pharmaceutical Dosage Forms, 2nd Ed. (various editors, 1989-1998, Marcel Dekker); and Pharmaceutical Dosage Forms and Drug Delivery Systems (Ansel et al., 1994, Williams & Wilkins).

Good manufacturing practices are known in the pharmaceutical industry and regulated by government agencies (e.g., Food and Drug Administration). A liquid formulation may be prepared by dissolving an intended component of the formulation in a sufficient amount of an appropriate solvent. Generally, dispersions are prepared by incorporating the various components of the formulation into a vehicle which contains the dispersion medium. For production of a solid form from a liquid formulation, solvent may be evaporated at room temperature or in an oven. Blowing a stream of nitrogen or air over the surface accelerates drying; alternatively, vacuum drying or freeze drying can be used. Solid dosage forms (e.g., powders, granules, pellets, tablets), liquid dosage forms (e.g., liquid in ampules, capsules, vials), and patches can be made from at least one active ingredient or component of the formulation.

Suitable procedures for making the various dosage forms and production of patches are known. The formulation may also be produced by encapsulating solid or liquid forms of at least one active ingredient or component, or keeping them separate in compartments or chambers. The patch may include a compartment containing a vehicle (e.g., saline solution) which is disrupted by pressure and subsequently solubilizes the dry formulation of the patch. The size of each dose and the interval of dosing to the subject may be used to determine a suitable size and shape of the container, compartment, or chamber.

Formulations will contain an effective amount of the active ingredients (e.g., at least one adjuvant and/or one or more antigens) together with carrier or suitable amounts of vehicle in order to provide pharmaceutically-acceptable compositions suitable for administration to a human or animal. Formulation that include a vehicle may be in the form of a cream, emulsion, gel, lotion, ointment, paste, solution, suspension, or other liquid forms known in the art; especially those that enhance skin hydration. For a patch, successive coatings of formulation may be applied to the substrate or several formulation-containing layers may be laminated to increase its capacity for active ingredients.

The relative amounts of active ingredients within a dose and the dosing schedule may be adjusted appropriately for efficacious administration to a subject (e.g., animal or human). This adjustment may depend on the subject's particular disease or condition, and whether therapy or prophylaxis is intended. To simplify administration of the formulation to the subject, each unit dose would contain the active ingredients in predetermined amounts for a single round of immunization.

There are numerous causes of protein instability or degradation, including hydrolysis and denaturation. In the case of denaturation, the protein's conformation is disturbed and the protein may unfold from its usual globular structure. Rather than refolding to its natural conformation, hydrophobic interaction may cause clumping of molecules together *(i.e.,* aggregation) or refolding to an unnatural conformation. Either of these results may entail diminution or loss of antigenic or adjuvant activity. Stabilizers may be added to lessen or prevent such problems.

The formulation, or any intermediate in its production, may be pretreated with protective agents (*i.e*., cryoprotectants and dry stabilizers) and then subjected to cooling rates and final temperatures that minimize ice crystal formation. By proper selection of cryoprotective agents and the use of preselected drying parameters, almost any formulation might be cryoprepared for a suitable desired end use.

It should be understood in the following discussion of optional additives like binders, buffers, colorings, dessicants, diluents, humectants, preservatives, and stabilizers are described by their function. Thus, a particular chemical may act as some combination of the aforementioned. Such chemicals would be considered immunologically-inactive because they do not directly induce an immune response, but it increases the response by enhancing immunological activity of the antigen or adjuvant: for example, by reducing modification of the antigen or adjuvant, or denaturation during drying and hydrating cycles.

Stabilizers include dextrans and dextrins; glycols, alkylene glycols, polyalkane glycols, and polyalkylene glycols, sugars and starches, and derivatives thereof are suitable. Preferred additives are nonreducing sugars and polyols. In particular, glycerol, trehalose, hydroxymethyl or hydroxyethyl cellulose, ethylene or propylene glycol, trimethyl glycol, vinyl pyrrolidone, and polymers thereof may be added. Alkali metal salts, ammonium sulfate, magnesium chloride, and surfactants (e.g., nonionic detergent), may stabilize proteinaceous adjuvants or antigens; optionally adding a carrier (e.g., agar, albumin, gelatin, glycogen, heparin), and freeze drying may further enhance stability. A polypeptide may also be stabilized by contacting it with a sugar such as, for example, a monosaccharide, disaccharide, sugar alcohol, and mixtures thereof (e.g., arabinose, fructose, galactose, glucose, lactose, maltose, mannitol, mannose, sorbitol, sucrose, xylitol). Polyols may stabilize a polypeptide, and are water-miscible or water-soluble. Various other excipients may also stabilize polpeptides, including amino acids, fatty acids and phospholipids, metals, reducing agents, and metal chelating agents.

Single-dose formulations can be stabilized in poly(lactic acid) (PLA) and poly (lactide-co-glycolide) (PLGA) microspheres by suitable choice of stabilizer or other excipients. Trehalose may be advantageously used as an additive because it is a nonreducing saccharide, and therefore does not cause aminocarbonyl reactions with substances bearing amino groups such as proteins. Although stabilizers like high concentrations of sugar will combat the growth of microbes like bacteria and fungi, preservatives are typically antimicrobial agents that actively eliminate (e.g., bacteriocidal) or reduce the growth of microbes (e.g., bacteriostatic). Antioxidants may also be used to prevent oxidation of active ingredients of the formulation.

It is conceivable that a formulation or patch that can be administered to the subject in a dry, nonliquid *(i.e.,* solid) form, may allow storage in conditions that do not require a cold chain. An antigen may be mixed with a heterologous adjuvant, placed on a dressing to form a patch, and allowed to completely dry. This dry patch can then be placed on skin with the dressing in direct contact with the skin for a period of time and be held in place covered with an occlusive backing layer (e.g., plastic or wax film).

Patch material may be nonwoven or woven (e.g., gauze dressing). Layers may also be laminated during processing. It may be nonocclusive or occlusive, but the latter is preferred for backing layers. The optional release liner preferably does not adsorb significant amounts of the formulation, perhaps by modifying a film with silicone- or fluoro-type agents. The patch is preferably hermetically sealed for storage (e.g., foil packaging). The patch can be held onto the skin and components of the patch can be held together using various adhesives. One or more of the adjuvant and/or antigen may be incorporated into the substrate or adhesive parts of the patch. Generally, patches are planar and pliable, and they are manufactured with a uniform shape. Optional additives are plasticizers to maintain pliability of the patch, tackifiers to assist in adhesion between patch and skin, and thickeners to increase the viscosity of the formulation at least during processing.

Metal foil, cellulose, cloth (e.g., acetate, cotton, rayon), acrylic polymer, ethylenevinyl acetate copolymer, polyamide (e.g., nylon), polyester (e.g., polyethylene naphthalate, ethylene terephthalate), polyolefin (e.g., polyethylene, polypropylene), polyurethane, polyvinyl alcohol, polyvinyl pyrrolidone, polyvinylidene chloride (SARAN), natural or synthetic rubber, silicone elastomer, and combinations thereof are examples of patch materials (e.g., dressing, backing layer, release liner).

The adhesive may be an aqueous-based adhesive (e.g., acrylate or silicone). Acrylic adhesives are available from several commercial sources. Acrylic polymers may be a copolymer of C4-C18 aliphatic alcohol with methacrylic alkyl ester or the copolymer of methacrylic alkyl ester having C4-C18 alkyl, methacrylic acid, and/or other functional monomers. Examples of the methacrylic alkyl ester may include butyl acrylate, isobutyl acrylate, hexyl acrylate, octyl acrylate, 2-ethylhexyl acrylate, iso-octyl acrylate, decyl acrylate, isodecyl acrylate, lauryl acrylate, stearyl acrylate, methyl methacrylate, ethyl methacrylate, butyl methacrylate, isobutyl methacrylate, 2-ethylhexyl methacrylate, iso-octyl methacrylate, decyl methacrylate, etc.

Examples of the functional monomers may include a monomer containing hydroxyl group, a monomer containing carboxyl group, a monomer containing amide group, a monomer containing amino group. The monomer containing hydroxyl group may include hydroxyalkyl methacrylate such as 2-hydroxyethyl methacrylate, hydroxypropyl methacrylate and the like. The monomer containing carboxyl group may include a-β unsaturated carboxylic acid such as acrylic acid, methacrylic acid and the like; maleic mono alkyl ester such as butyl malate and the like; maleic acid; fumaric acid; crotonic acid and the like; and anhydrous maleic acid. Examples of the monomer containing amide group may include alkyl methacrylamide such as acrylamide, dimethyl acrylamide, diethyl acrylamide and the like; alkylethylmethylol methacrylamide such as butoxymethyl acrylamide, ethoxymethyl acrylamide and the like; diacetone acrylamide; vinyl pyrrolidone; dimethyl aminoacrylate. In addition to the above exemplified monomers for copolymerization, vinyl acetate, styrene, α-methylstyrene, vinyl chloride, acrylonitrile, ethylene, propylene, butadiene and the like may be employed.

Commercially available acrylic adhesives are sold under the tradenames AROSET, DUROTAK, EUDRAGIT, GELVA, and NEOCRYL. EUDRAGIT polymers form a diverse family of polymers whose common feature is a polyacrylic or polymethacrylic backbone that is compatible with the gastrointestinal tract and which have been widely used in pharmaceutical preparations, especially as coatings for tablets, but it has also been used as a coating for other medical devices. EUDRAGIT polymers are characterized as (1) an anionic copolymer based on methacrylic acid and methylmethacrylate wherein the ratio of free carboxyl groups to the ester groups is approximately 1:1, (2) an anionic copolymer based on methacrylic acid and methylmethacrylate wherein the ratio of free carboxyl groups to the ester groups is approximately 1:2, (3) a copolymer based on acrylic and methacrylic acid esters with a low content of quaternary ammonium groups wherein the molar ratio of the ammonium groups to the remaining neutral methacrylic acid esters is 1:20, and (4) a copolymer based on acrylic and methacrylic acid esters with a low content of quarternary ammonium groups wherein the molar ratio of the ammonium groups to the remaining neutral methacrylic acid esters is 1:40. The copolymers are sold under tradenames EUDRAGIT L, EUDRAGIT S, EUDRAGIT RL, and EUDRAGIT RS. EUDRAGIT E is a cationic copolymer based on diethylaminoethyl methacrylate and neutral methacrylic acid esters; EUDRAGIT NE is a neutral copolymer of polymethacrylates. For methacrylate or acrylate polymers, there are EUDRAGIT RS, EUDRAGIT RL, and EUDRAGIT NE; also available are EUDRAGIT RS-100, EUDRAGIT L-90, EUDRAGIT NE-30, EUDRAGIT L-100, EUDRAGIT S-100, EUDRAGIT E-100, EUDRAGIT RL-100, EUDRAGIT RS-100, EUDRAGIT RS-30D, EUDRAGIT E-100R, and EUDRAGIT RTM.

Furthermore, for the purpose of increasing or decreasing the water absorption capacity of an adhesive layer, the acrylic polymer may be copolymerized with hydrophilic monomer, monomer containing carboxyl group, monomer containing amide group, monomer containing amino group, and the like. Rubbery or silicone resins may be employed as the adhesive resin; they may be incorporated into the adhesive layer with a tackifying agent or other additives.

Alternatively, the water absorption capacity of the adhesive layer can be also regulated by incorporating therein highly water-absorptive polymers, polyols, and water-absorptive inorganic materials. Examples of the highly water-absorptive resins may include mucopolysaccharides such as hyaluronic acid, chondroitin sulfate, dermatan sulfate and the like; polymers having a large number of hydrophilic groups in the molecule such as chitin, chitin derivatives, starch and carboxy-methylcellulose; and highly water-absorptive polymers such as polyacrylic, polyoxyethylene, polyvinyl alcohol, and polyacrylonitrile. Examples of the water-absorptive inorganic materials, which may incorporated into the adhesive layer to regulate its water absorptive capacity, may include powdered silica, zeolite, powdered ceramics, and the like.

The plasticizer may be a trialkyl citrate such as, for example, acetyl-tributyl citrate (ATBC), acetyl-triethyl citrate (ATEC), and triethyl citrate (TEC). The plasticizer may be between 0.001 % (w/v) and 5% (w/v) of the adhesive formulation. A suitable concen-tration may be empirically determined by selecting for pliability of the adhesive layer, and avoiding brittleness.

Exemplary tackifiers are glycols (e.g., glycerol, 1,3 butanediol, propylene glycol, polyethylene glycol); average molecular weights of 200, 300, 400, 800, 3000, etc. are available for the polyakylene glycols. Succinic acid is another tackifier. The tackifier may be between 0.1% (w/w) and 10% (w/w) of the adhesive formulation. A suitable concentration may be empirically determined by avoiding brittleness of the adhesive layer and its pliability.

Thickeners can be added to increase the viscosity of an adhesive or immunogenic formulation. The thickener may be a hydroxyalkyl cellulose or starch, or water-soluble polymers: for example, poloxamers, polyethylene oxides and derivatives thereof, polyethyleneimines, polyethylene glycols, and polyethylene glycol esters. But any molecule which serves to increase the viscosity of a solution may be suitable to improve handling of a formulation during manufacture of a patch. For example, hydroxyethyl or hydroxypropyl cellulose may be between 1% (w/w) and 10% (w/w) of the adhesive or immunogenic formulation. The formulation as a layer may be film cast or extruded, and then layers may be coated or laminated during manufacture of a patch. The capacity for protein might be increased by successive coatings or laminating several thin, adhesive layers together. Alternatively, a viscous formulation may be spread on a substrate (e.g., backing or adhesive layer) with minimal loss of immunologically-active ingredients like adjuvant or antigen. Thickeners are sold as NATROSOL hydroxyethyl cellulose and KLUCEL hydroxypropyl cellulose.

Gel and emulsion systems can be incorporated into patch delivery systems, or be manufactured separately from the patch, or added to the patch prior to application to the human or animal subject. Gels or emulsions may serve the same purpose of facilitating manufacture by providing a viscous formulation that can be easily manipulated with minimal loss. The term "gel" refers to covalently crosslinked, noncrosslinked hydrogel matrices. Hydrogels can be formulated with at least one protein with immunologic activity for PIA patches. Additional excipients may be added to the gel systems that allow for the enhancement of antigen/adjuvant delivery, skin hydration, and protein stability. The term "emulsion" refers to formulations such as water-in-oil creams, oil-in-water creams, ointments, and lotions. Emulsion systems can be either micelle-based, lipid vesicle-based, or both micelle- and lipid vesicle-based. Emulsion systems can be formulated with at least one adjuvant and/or antigen as the protein-in-adhesive systems. Additional excipients may be added to the emulsion systems that allow for the enhancement of antigen/adjuvant delivery, skin hydration, and protein stability.

A liquid or quasi-liquid formulation may be applied directly to the skin and allowed to air dry; rubbed into the skin or scalp; placed on the ear, inguinal, or intertriginous regions, especially in animals; placed on the anal/rectal tissues; held in place with a dressing, patch, or absorbent material; immersion; otherwise held by a device such as a stocking, slipper, glove, or shirt; or sprayed onto the skin to maximize contact with the skin. The formulation may be applied in an absorbent dressing or gauze. The formulation may be covered with an occlusive dressing such as, for example, AQUAPHOR (an emulsion of petrolatum, mineral oil, mineral wax, wool wax, panthenol, bisabol, and glycerin from Beiersdorf), plastic film, COMFEEL (Coloplast) or VASELINE petroleum jelly; or a nonocclusive dressing such as, for example, TEGADERM (3M), DUODERM (3M) or OPSITE (Smith & Napheu). An occlusive dressing excludes the passage of water. Such a formulation may be applied to single or multiple sites, to single or multiple limbs, or to large surface areas of the skin by complete immersion. The formulation may be applied directly to the skin. Other substrates that may be used are pressure-sensitive adhesives such as acrylics, polyisobutylenes, and silicones. The formulation may be incorporated directly into such substrates, perhaps with the adhesive *per se* instead of adsorption to a porous pad (e.g., cotton gauze) or bilious strip (e.g., cellulose paper).

The adhesive and immunogenic formulations may be at least partially mixed or even throroughly blended, and then adhered to the backing layer. The immunologically-active ingredient may be dispersed or dissolved in the formulation. Adhesive may be brought into contact with a release liner. Adhesive and immunogenic formulations may also be brought into contact with microblade or microneedle arrays or tines by coating, dipping the device into the formulation and drying, or spraying the device with the formulation. formulations may be at least partially mixed or even throroughly blended, and then adhered to the backing layer. The immunologically-active ingredient may be dispersed or dissolved in the formulation. Alternatively the immunogenic formulation may be applied to the surface of the adhesive layer by coating or spreading over the adhesive using a Meyer rod, casting a layer and then laminating in close apposition with the adhesive using a roller, printing on the adhesive using a rotogravure, etc.

Polymers added to the formulation may act as a stabilizer or other excipient of an active ingredient as well as reducing the concentration of the active ingredient that saturates a solution used to hydrate an at least partially-dried form *(i.e.,* dry or semi-liquid) of the active ingredient. Such reduction occurs because the polymer reduces the effective free volume by filling "empty" space in the solvent. In this way, quantities of adjuvant/antigen can be conserved without reducing the amount of saturated solution. An important thermodynamic consideration is that an active ingredient in the saturated solution will be "driven" into regions of lower concentration (e.g., through the skin). For dispersal or dissolution of at least one adjuvant and/or one or more antigens, polymers can also stabilize the adjuvant/antigen-activity of those components of the formulation. Such polymers include ethylene or propylene glycol, vinyl pyrrolidone, and β-cyclodextrin polymers and copolymers.

### TRANSCUTANEOUS DELIVERY

Transcutaneous delivery of the formulation may target Langerhans cells and, thus, achieve effective and efficient immunization. These cells are found in abundance in the skin and are efficient antigen presenting cells (APC), which can lead to T-cell memory and potent immune responses. Because of the presence of large numbers of Langerhans cells in the skin, the efficiency of transcutaneous delivery may be related to the surface area exposed to antigen and adjuvant. In fact, the reason that transcutaneous immunization is so efficient may be that it targets a larger number of these efficient antigen presenting cells than intramuscular immunization.

Immunization may be achieved using epicutaneous application of a simple formulation of antigen and adjuvant, optionally covered by an occlusive dressing or using other patch technologies, to intact skin with or without chemical or physical penetration. Transcutaneous immunization according to the invention may provide a method whereby antigens and adjuvant can be delivered to the immune system, especially specialized antigen presentation cells underlying the skin (e.g., dendritic cells like Langerhans cells).

For traditional vaccines, their formulations were injected through the skin with needles. Injection of vaccines using needles carries certain drawbacks including the need for sterile needles and syringes, trained medical personnel to administer the vaccine, discomfort from the injection, needle-born diseases, and potential complications brought about by puncturing the skin with the potentially reusable needles. Immunization through the skin without the use of hypodermic needles represents an advance for vaccine delivery by avoiding the hypodermic needles.

Moreover, transcutaneous immunization may be superior to immunization using hypodermic needles as more immune cells would be targeted by the use of several locations targeting large surface areas of skin. A therapeutically-effective amount of antigen sufficient to induce an immune response may be delivered transcutaneously either at a single cutaneous location, or over an area of skin covering multiple draining lymph node fields (e.g., cervical, axillary, inguinal, epitrochelear, popliteal, those of the abdomen and thorax). Such locations close to numerous different lymphatic nodes at locations all over the body will provide a more widespread stimulus to the immune system than when a small amount of antigen is injected at a single location by intradermal, subcutaneous, or intramuscular injection.

Antigen passing through or into the skin may encounter antigen presenting cells which process the antigen in a way that induces an immune response. Multiple immunization sites may recruit a greater number of antigen presenting cells and the larger population of antigen presenting cells that were recruited would result in greater induction of the immune response. It is conceivable that use of the skin may deliver antigen to phagocytic cells of the skin such as, for example, dendritic cells, Langerhans cells, macrophages, and other skin antigen presenting cells; antigen may also be delivered to phagocytic cells of the liver, spleen, and bone marrow that are known to serve as the antigen presenting cells through the blood stream or lymphatic system.

Langerhans cells, other dendritic cells, macrophages, or combinations thereof may be specifically targeted using their asialoglycoprotein receptor, mannose receptor, Fcγ receptor CD64, high-affinity receptor for IgE, or other highly expressed membrane proteins. A ligand or antibody specific for any of those receptors may be conjugated to or recombinantly produced as a protein fusion with adjuvant, antigen, or both. Furthermore, adjuvant, antigen, or both may be conjugated to or recombinantly produced as a protein fusion with protein A or protein G to target surface immunoglobulin of B lymphocytes. The envisioned result would be widespread distribution of antigen to antigen presenting cells to a degree that is rarely, if ever achieved, by current immunization practices.

A specific immune response may comprise humoral (*i.e.,* antigen-specific antibody) and/or cellular (*i.e*., antigen-specific lymphocytes such as B lymphocytes, CD4⁺ T cells, CD8⁺ T cells, CTL, Th1 cells, Th2 cells, and/or T_{DTH} cells) effector arms. Moreover, the immune response may comprise NK cells and other leukocytes that mediate antibody-dependent cell-mediated cytotoxicity (ADCC).

The immune response induced by the formulation of the invention may include the elicitation of antigen-specific antibodies and/or lymphocytes. Antibody can be detected by immunoassay techniques. Detection of the various antibody isotypes (e.g., IgM, IgD, IgA1, IgA2, secretory IgA, IgE, IgG1, IgG2, IgG3 or IgG4) can be indicative of a systemic or regional immune response. Immune responses can also be detected by a neutralizing assay. Antibodies are protective proteins produced by B lymphocytes. They are highly specific, generally targeting one epitope of an antigen. Immunization may induce antibodies that neutralize biological activity of an allergen, cell-entry receptor, growth factor receptor, or toxin. For example, inducing antibodies may treat a disease by specifically reacting with antigen (e.g., cholera toxin, HER2, influenza hemagluttinin) derived from a pathogen or cancer. Challenge studies in a host using infection by the pathogen or administration of toxin, comparison of morbidity or mortality between immunized and control populations, or measurement of another clinical criterion (e.g., high antibody titers or production of IgA antibody-secreting cells in mucosal membranes may be used as a surrogate marker) can demonstrate protection against disease or therapy of existing disease.

CTL are immune cells produced to protect against infection by a pathogen. They are also highly specific. Immunization may induce CTL specific for the antigen in association with self-major histocompatibility complex antigen. CTL induced by immunization with the transcutaneous delivery system may kill pathogen-infected cells or cancers. Immunization may also produce a memory response as indicated by boosting responses in antibodies and CTL, proliferation of lymphocyte cultures stimulated with the antigen, and delayed-type hypersensitivity (DTH) responses to intradermal skin challenge of the antigen alone.

The following is meant to be illustrative of the invention, but practice of the invention is not limited or restricted in any way by the following examples.

### EXAMPLES

### MATERIALS AND METHODS

Vaccines. Tetanus toxoid and multivalent influenza vaccines were used for the purpose of exemplifying the invention. Animals receiving the tetanus toxoid vaccine were vaccinated by splitting the dose (0.5 LF) volume between both thigh muscles. The influenza vaccine consisted of three strains originally isolated from humans (Panama A strain/2007/99, Johannesburg B strain/15/99 and New Caledonia/20/99). The vaccine was prepared as an admixture of equal protein mass of each strain (trivalent flu vaccine). In one experiment, the influenza vaccine was injected by splitting the dose (5 µg of trivalent flu) between both thigh muscles. In a separate experiment, animals received 1.5 µg of trivalent influenza by subcutaneous injection into the dorsal caudal surface at the base of the tail. All animals were parenterally immunized immediately prior to application of the adjuvant-containing patch.

LT-containing patch. Mice were shaved on the dorsal caudal surface at the base of the tail (24 hr to 48 hr) prior to vaccination. All mice were anesthetized by intraperitoneal injection of 25 µl of a mixture of ketamine (100 mg/ml) and xylazine (100 mg/ml). The shaved skin was pretreated by hydration with water or an aqueous solution of 10% glycerol, 70% isopropyl alcohol, and 20% water. Immediately prior to application, a gauze pad (about 1 cm²), which was affixed on an adhesive backing, was loaded with 25 µl of a solution containing either 10 µg or 50 µg of LT formulated in neutral phosphate buffered saline and 5% lactose. The patch was applied to the hydrated skin surface for 1 hr, the patch removed, and the skin rinsed with water to remove excess LT.

Vaccination regimen. In studies with tetanous toxoid, mice received three intramuscular injections (with a patch) on study days 0, 14 and 28. Serum was collected 14 days after the third immunization (day 49). Two vaccination regimens were used to immunize mice with the trivalent influenza vaccine: (i) two intramuscular injections (with a LT patch) on days 0 and 14 of the study, with serum collection two weeks after the second vaccination (day 28) or (ii) three intramuscular or subcutaneous injections (with a LT patch) on days 0, 14 and 28 of the study, with serum collection two weeks after the third dose (day 42).

Sample collection. Peripheral blood was obtained by lacerating the tail vein. The blood was collected in a tube, allowed to clot, and centrifuged. The serum was collected and then stored at -20°C until assayed by the ELISA method.

ELISA method. A solid-phase enzyme-linked immunosorbent assay method was used to assess the serum IgG. Ninety-six well plates were coated with 100 µl (1-2 µg antigen) per well overnight at 4°C. For influenza, ELISA plates were coated separately with antigen from each of the influenza stains used in the trivalent vaccine (*i.e*., Panama A, New Caledonia A, and Johannesburg B strains). After washing with phosphate buffered saline and Tween 20 (PT buffer), the plates were blocked with 100 µl of blocking buffer (0.5% casein and 0.5% bovine serum albumin) for 1 hr at room temperature. The plates were washed with PT buffer and the serum was twofold serially diluted in the wells. The plates were incubated overnight at 4°C. The plates were washed with PT buffer and 100 µl of 1:2000 diluted goat anti-mouse IgG conjugated with HRP (BioRad) as added to each well. The plates were incubated for 2 hours at room temperature, washed with PT buffer, and 100 µl of substrate ABTS (KPL) was added to the wells. The color was allowed to develop for about 30 min. The reaction was stopped by adding 100 µl of 1% SDS solution (GIBCO BRL). The optical density was measured at 405 nm with an ELISA plate reader and the data analyzed using Softmax Pro 2.4 software (Molecular Devices). The results are expressed in ELISA Units (EU), which is the serum dilution that resulted in an optical density (OD) reading equal to 1 OD at 405 nm. The EU for each serum sample was plotted with the geometric mean titer (GMT) for the group (Bar) indicated in each figure.

### EXAMPLE 1: Systemic immunostimulation elicited by adjuvant in mice that were vaccinated with low doses of tetanus toxoid in the thigh muscles

It was determined whether epicutaneous application of adjuvant will stimulate the immune response induced by low doses of tetanus toxoid that are administered within skeletal muscle, a standard route of vaccination. In this study, all mice were vaccinated with tetanus toxoid in the thigh muscle. Immediately following vaccination, the shaved skin surface at the base of the tail was hydrated with 10% glycerol, 70% isopropyl alcohol, and 20% water. Half of the vaccinated mice received a gauze patch (on an adhesive backing) that contained 50 µg of LT in a solution (PBS with 5% lactose). The patch was applied to the hydrated skin for 1hr. The patch was removed and the skin was washed. In this study, mice received three vaccinations (and patches) on study days 0, 14 and 28. The serum was collected three weeks after the third vaccination and the antibody titers to tetanus toxoid determined for both groups of animals. Figure 1 demonstrates two important points about the effect that the LT-containing transcutaneous patch had on the immune response to the vaccine. Firstly, animals that received the LT patch exhibit a statistically significant (p>0.005) increase in antibody titer (GMT = 1:74,155) compared to the group that did not receive the LT patch (GMT = 1:10,196). Secondly, the group of animals with the LT patch not only exhibited a greater magnitude in serum antibody response, but the titers tended to be tightly clustered around (1:74,000) compared to a wide spread (1:500 to 1:100,000) of titers within the group that was intramuscularly vaccinated without a LT patch. These results indicate that LT uniformly stimulated the immune response in a population that otherwise exhibited a heterogeneous response including animals that poorly respond to the vaccine. This suggests that LT-mediated immunostimulation may be able to positively affect even poor immune responders such as immunocompromised subjects.

### EXAMPLE 2: Comparison of the effect of adjuvant-containing patches on stimulating the immune response to trivalent influenza vaccine which is parenterally administered either in skeletal muscle or subcutaneous tissue

It was next determined whether the tissue location of the influenza vaccination affects the immunostimulating activity of epicutaneously applied adjuvant, and if the LT dose could be decreased from 50 µg to 10 µg in a patch. Groups of mice were vaccinated by intramuscular injection of 5 µg of trivalent influenza (1.7 µg per strain) vaccine split between both thigh muscles. Immediately following injection, the shaved skin surface at the base of the tail was hydrated with water. Patches were applied to the hydrated skin. One-third of the mice received a patch containing phosphate buffered saline (PBS, vehicle control); one-third received a patch containing 10 µg of LT; and one-third received a patch loaded with 50 µg of LT. The patches were applied for 1 hr, removed, and the skin rinsed to remove excess LT. All mice were vaccinated twice (study days 0 and 14) and the serum was collected two weeks after the second immunization and patch. The serum antibody titers to the Panama A strain were determined using the ELISA method. The results are depicted in Figure 2A. As seen in this figure, the group with the 50 µg LT patches developed very high serum antibody titers to influenza antigens (GMT = 1:276,485) that were significantly greater (p = 0.005) than the group with the negative control patch (GMT = 1:40,531). The group with the 10 µg LT patches tended to have higher titers to influenza vaccine (GMT = 1:67,617) compared to the negative control group (p = 0.192).

Another study was performed to determine if the influenza vaccine could be placed into the subcutaneous tissues with an LT patch overlayed over the vaccination site. In this study, all mice were vaccinated with a very low dose (1.5 µg) of trivalent influenza vaccine (0.5 µg of each strain). A subcutaneous injection was administered in the shaved dorsal caudal surface at the base of the tail, which had been hydrated with water prior to injection. Several minutes after the injection, a patch was applied over the injection site. One-third of the mice received a patch containing PBS (negative control), one-third received a patch containing 10 µg LT, and one-third received a patch loaded with 50 µg LT. The patches were applied for 1 hr, removed, and the skin rinsed to remove the excess LT. All mice were vaccinated twice (with patches) on study days 0 and 14. The serum was collected two weeks after the second immunization (day 28). Serum IgG titers to the Panama A strain were determined using the ELISA method. These results are illustrated in Figure 2B. Again, mice receiving the 50 µg LT patch exhibited a significantly (p = 0.001) greater antibody titer (GMT = 1:111,434) compared to the group that received the negative control patch (GMT = 11,793). The group that received the 10 µg LT patch had a 5-fold greater titer to influenza vaccine (GMT = 52,606) compared to the group receiving the negative control patches (p = 0.08).

Epicutaneous application of an adjuvant-containing patch significantly stimulated the immune response to an inactivated, trivalent influenza vaccine. The site of the vaccination did not appear to affect the immunostimulating action of the epicutaneously applied LT. Adjuvant stimulated the immune response whether the vaccine was injected into skeletal muscle or into subcutaneous tissues. These two studies also demonstrate that very low doses of influenza vaccine (5 µg trivalent in the muscle or 1.5 µg trivalent subcutaneous) may be administered and significant antibody titers elicited (>1:100,000) by transcutaneous delivery of adjuvant.

### EXAMPLE 3: Adjuvant-containing patch stimulates the immune response to a multivalent influenza vaccine that is injected into skeletal muscle

The purpose of this study was to demonstrate that the LT patch did stimulate immune responses to each strain of influenza in the trivalent vaccine. In this study, all mice were vaccinated by injection of low dose (5 µg) trivalent influenza vaccine into both thigh muscles. Immediately following injection, the shaved skin surface (shaved 48 hr prior) at the base of the tail was hydrated with water. To half of the mice, a patch loaded with 50 µg LT was applied to the hydrated skin. The other half did not receive an LT patch. As in the other studies, the patches were applied to the skin for 1 hour, removed and the skin rinsed to remove the excess LT. Both groups were vaccinated with influenza vaccine three times (days 0, 14 and 28). Serum was collected two weeks after the third immunization and antibody titers to each strain of influenza in the vaccine determined. The group receiving the LT patch with intramuscular injection of flu vaccine exhibited a 3-fold to 4-fold greater titer than did the group receiving just the intramuscular injection (Figure 3). In addition, this also demonstrates that the LT patch stimulated the systemic immune response to all three strains of influenza in the vaccine (Panama, Fig. 3A; Johannesburg, Fig. 3B; and New Caledonia, Fig. 3C).

It may be important that the same region of the lymph system be targeted by the adjuvant delivered transcutaneously and the antigen delivered by a route other than transcutaneous. Migration of Langerhans cells and other skin dendritic cells was followed by applying fluorescein isothiocyanate (FITC) epicutaneously to skin. Antigen presenting cells (APC) are phagocytic and took up FITC before migrating out of the skin and to a regional lymph node. A fluorescence activated cell analyzer was used to immunophenotype cells of the lymph node and to detect FITC phagocytosed by the APC. CD11b-antibody stained APC. Activated APC were detected by their up-regulation of MHC Class II molecules and co-stimulatory molecules using labeled antibodies. The adjuvant may have activated APC underlying the skin where the transcutaneous immunization occurred. Both FITC and adjuvant were applied to the same site on the skin.

A kinetic study showed that APC trafficked to nearby lymph nodes after the adjuvant was epicutaneously applied to the skin: FITC-labeled APC began to be detected at about 7 hr after immunostimulation, their abundance reached its peak at about 24 hr, the numbers of FITC-labeled APC started to fall about 48 hr after immunostimulation, and FITC-labeled APC were not detected after about 72 hr.

Localization of migrating APC showed that those underlying the skin traffic to the same regional lymph node. FITC and adjuvant applied on the back and base of the animal's tail caused APC to migrate to a proximal, inguinal lymph node. APC were not seen to migrate to a distal, cervical lymph node after adjuvant was applied on the back and base of the animal's tail. In contrast, FITC and adjuvant applied on the neck had the opposite trafficking pattern: APC migrated to a proximal, cervical lymph node instead of a distal, inguinal lymph node.

Therefore, it is preferred that immunization with adjuvant (*i.e.,* transcutaneous immunostimulation) and immunization with vaccine (*i.e.,* vaccination) occur within a few hours to a day instead of more than 72 hours apart. Moreover, trafficking of APC to regional lymph nodes shows that the sites at which transcutaneous immunostimulation and vaccination occur should be close enough together that APC will migrate to at least one shared lymph node.

Using two different model vaccines, these examples demonstrate that an epicutaneously applied adjuvant-containing patch stimulates the immune response to a vaccine administered by a different route. The toxicity associated with administration of an adjuvant through other routes (e.g., enteric, mucosal, injected into the circulation) did not occur with transcutaneous immunization. These results demonstrate that the use of epicutaneously applied adjuvant is safe and effective in stimulating an effective immune response.

### EXAMPLE 4: Protein-in-adhesive and air-dried patch formulations for LT-containing patch

A patch provides a versatile device for delivery of adjuvant by epicutaneous appli-cation to skin. Here, LT was formulated using four different patch configurations. For a first formulation, LT (10 µg) was formulated in an aqueous solution consisting of neutral pH phosphate buffered saline containing 5% (w/v) lactose. This formulation was applied directly to skin that was hydrated with 10% glycerol, 70% isopropyl alcohol, and 20% water. The solution was left undisturbed or was overlaid with a gauze pad for 1 hr. For a second formulation, LT was blended with a pressure-sensitive EUDRAGIT EPO adhesive, KLUCEL thickener, and a stabilizer of 1 % sucrose. The formulation was then spread as a thin coat over an occlusive backing. The LT was spread with a rotograveur press as a fine film to an effective concentration of 10 µg/cm² area. The film was air dried at room temperature and moisture content ranged between < 0.2% to 5% water. Patches (about 1 cm²) were punched from the sheet. The at least partially dried patches were stored at ambient temperature and 4°C exhibited the same delivery characteristics. For a third formulation, LT was directly applied to a NU-GAUZE pad, spread evenly over the surface to a concentration of 10 µg/cm², and the at least partially dry patch was air dried overnight. For a fourth formulation, the LT (10 µg in 25 µl PBS and 5% lactose) in an aqueous formulation was dropped directly onto a gauze pad (about 1 cm²) that was affixed to an adhesive backing. These patches were air dried at ambient temperature overnight. At least partially dried patches can be stored at 4°C or room temperature for one month or longer prior to use.

The patches were compared for delivery of LT antigen using the mouse model described above. Here, the shaved skin at the base of the tail was hydrated and pretreated with a pumice-containing swab (a 10% glycerol, 70% isopropyl alcohol, and 20% water solution for water) to disrupt the stratum corneum. Groups of 5 mice received a first patch on day 0 and a second patch on day 14. The air dried patch was rehydrated with 25 µl of water prior to application. The patches were removed after 24 hr. For the liquid formulation, the LT containing solution was left on the skin for 1 hr prior to rinsing with water to remove excess LT. Serum was collected from each animal two weeks after the second immunization (day 28). These results demon-strate that all methods were suitable for delivery of LT via the skin surface. This example shows that the patch formula may be an aqueous liquid that is applied directly to skin and over laid with a patch; a dry patch with the LT incorporated within the adhesive (protein-in-adhesive) and spread as a thin coating over an occlusive backing; a patch in which the LT is applied as a solution directly to a suitable surface and allowed to air dry; or as a hydrated patch in which the appropriate amount of the LT solution is directly applied to patch surface shortly before applying the patch to the skin.

All references (e.g., articles, books, patents, and patent applications) cited above are indicative of the level of skill in the art and are incorporated by reference.

All modifications and substitutions that come within the meaning of the claims and the range of their legal equivalents are to be embraced within their scope. A claim using the transition "comprising" allows the inclusion of other elements to be within the scope of the claim; the invention is also described by such claims using the transitional phrase "consisting essentially of' (*i.e.,* allowing the inclusion of other elements to be within the scope of the claim if they do not materially affect operation of the invention) and the transition "consisting" *(i.e.,* allowing only the elements listed in the claim other than impurities or inconsequential activities which are ordinarily associated with the invention) instead of the "comprising" term. No particular relationship between or among limitations of a claim is meant unless such relationship is explicitly recited in the claim (e.g., the arrangement of components in a product claim or order of steps in a method claim is not a limitation of the claim unless explicitly stated to be so). Thus, all possible combinations and permutations of the individual elements disclosed herein are intended to be considered part of the invention.

From the foregoing, it would be apparent to a person of skill in this art that the invention can be embodied in other specific forms without departing from its spirit or essential characteristics. The described embodiments should be considered only as illustrative, not restrictive, because the scope of the legal protection provided for the invention will be indicated by the appended claims rather than by this specification

## Claims

1. An adjuvant for transcutaneous immunostimulation comprising:
(a) providing a subject in need of immunization with a vaccine,
(b) applying at least one adjuvant epicutaneously to the subject's skin, and
(c) immunizing the subject with the vaccine by a route of administration other than transcutaneous, wherein the vaccine comprises one or more antigens;
whereby the at least one adjuvant causes transcutaneous immunostimulation by inducing an immune response specific for the one or more antigens, wherein the immune response stimulated by the at least one adjuvant is more effective than in the absence of the at least one adjuvant.

2. An adjuvant according to claim 1, wherein the subject is over 65 years old.

3. An adjuvant according to claim 1, wherein the subject is immunocompromised.

4. An adjuvant according to claim 1, wherein the subject is immunosuppressed.

5. An adjuvant according to claim 1, wherein the vaccine contains an amount of the one or more antigens which is not sufficient to induce the antigen-specific immune response without an adjuvant.

6. The adjuvant of claim 1, wherein the vaccine is administered orally.

7. The adjuvant of claim 1, wherein the vaccine is administered intranasally.

8. The adjuvant of claim 1, wherein the vaccine is administered by injection.

9. The adjuvant of claim 1, wherein the adjuvant activates an antigen presenting cell underlying the skin.

10. The adjuvant of claim 1, provided in dry form.

11. The adjuvant of claim 1, provided as part of a patch.

12. The adjuvant of claim 1, wherein the subject's skin is hydrated.

13. The adjuvant of claim 1, wherein penetration of the subject's skin by the at least one adjuvant is enhanced with one or more chemical agents and/or physical disruption devices.

14. The adjuvant of claim 1, wherein the vaccine lacks an adjuvant.

15. The adjuvant of claim 12, wherein the vaccine further comprises at least one adjuvant.
